(19) Europäisches Patentamt
European Patent Office
Office européen des brevets

(11) Veröffentlichungsnummer : **0 185 256 B1**

# (12) EUROPÄISCHE PATENTSCHRIFT

(45) Veröffentlichungstag der Patentschrift : **30.01.91 Patentblatt 91/05**

(51) Int. Cl.$^5$ : **C07D 249/08**, C07D 401/04, A01N 43/653

(21) Anmeldenummer : **85115436.9**

(22) Anmeldetag : **05.12.85**

## (54) Triazolderivate, deren Herstellung und Verwendung als Schädlingsbekämpfungsmittel.

Die Akte enthält technische Angaben, die nach dem Eingang der Anmeldung eingereicht wurden und die nicht in dieser Patentschrift enthalten sind.

(30) Priorität : **21.12.84 CH 6105/84**
**25.09.85 CH 4151/85**

(43) Veröffentlichungstag der Anmeldung : **25.06.86 Patentblatt 86/26**

(45) Bekanntmachung des Hinweises auf die Patenterteilung : **30.01.91 Patentblatt 91/05**

(84) Benannte Vertragsstaaten : **AT BE CH DE FR GB IT LI NL SE**

(56) Entgegenhaltungen :
**EP-A- 0 036 711**
**EP-A- 0 050 219**
**EP-A- 0 084 136**
**US-A- 4 151 169**

(73) Patentinhaber : **F. HOFFMANN-LA ROCHE AG**
**Postfach 3255**
**CH-4002 Basel (CH)**

(72) Erfinder : **Lüthy, Christoph, Dr.**
**Gfennstrasse 43**
**CH-8603 Schwerzenbach (CH)**
Erfinder : **Zurflüh, René, Dr.**
**Dachslenbergstrasse 54**
**CH-8180 Bülach (CH)**

(74) Vertreter : **Urech, Peter, Dr. et al**
**Grenzacherstrasse 124 Postfach 3255**
**CH-4002 Basel (CH)**

**EP 0 185 256 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft heterocyclische Verbindungen, und zwar 1,2,4-Triazole der allgemeinen Formel

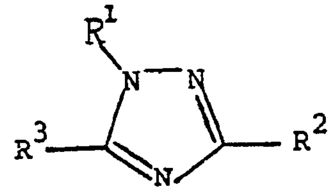

I

worin

$R^1$ $C_{1-4}$-Alkyl, $C_{1-4}$-Halogenalkyl oder $C_{2-4}$-Alkenyl,

$R^2$ mit 1 bis 3 Chlor-, Brom- und/oder Jodatomen, 1 bis 5 Fluoratomen, 1 oder 2 $C_{1-4}$-Alkylgruppen, 1 oder 2 Halogenmethylgruppen, 1 oder 2 $C_{1-2}$-Alkoxygruppen, 1 oder 2 $C_{1-2}$-Halogenalkoxygruppen, einer Methylthiogruppe, einer Cyanogruppe und/oder einer Nitrogruppe substituiertes Phenyl oder gegebenenfalls mit 1 bis 3 Chloratomen und/oder einer Methylgruppe substituiertes 2-, 3- oder 4-Pyridyl

und $R^3$ o-Trifluormethyl-phenyl oder 4-Trifluormethyl-3-pyridyl bedeuten,

sowie die Säureadditionssalze der Verbindungen der Formel I.

Die Verbindungen der Formel I und deren Säureadditionssalze sind Schädlingsbekämpfungsmittel und eignen sich insbesondere zur Bekämpfung von Insekten und Milben, z.B. Spinnmilben. Somit umfasst die Erfindung auch Schädlingsbekämpfungsmittel, welche Verbindungen der Formel I oder Säureadditionssalze davon als Wirkstoffe enthalten, ein Verfahren zur Herstellung dieser Verbindungen sowie die Verwendung dieser Verbindungen bzw. Mittel zur Bekämpfung von Schädlingen.

Auch die europäische Patentpublikation Nr. 36.711 offenbart 1-Alkyl-, 1-Halogenalkyl- sowie 1-Alkenyl-3,5-diaryl-1H-1,2,4-triazole und deren Verwendung als Pestizide. In den bekannten Verbindungen kann jedoch im Gegensatz zu den erfindungsgemässen Verbindungen weder der eine Arylsubstituent noch der andere Arylsubstituent o-Trifluormethyl-phenyl bzw. 4-Trifluormethyl-pyridyl sein.

Die in obiger Definition der Verbindungen der Formel I erwähnten $C_{1-4}$-Alkylreste und $C_{2-4}$-Alkenylreste können sowohl geradkettig als auch verzweigt sein. Dies gilt auch für den Alkylteil der $C_{1-4}$-Halogenalkylgruppe.

Der Ausdruck "Halogen" umfasst Fluor, Chlor, Brom und Jod. Die Gruppen "$C_{1-4}$-Halogenalkyl", "Halogenmethyl" und "$C_{1-2}$-Halogenalkoxy" können jeweils einen oder mehrere Halogensubstituenten aufweisen, die gleich oder verschieden sein können. Die Substituenten des Phenylrestes können ebenfalls gleich oder verschieden sein.

Durch das Vorliegen eines oder mehrerer asymmetrischer Kohlenstoffatome in den Verbindungen der Formel I treten die Verbindungen in optisch aktiver Form auf. Die Formel I soll demnach die Racemate sowie die aufgetrennten optisch aktiven Formen umfassen.

Als Säureadditionssalze der Verbindungen der Formel I kommen physiologisch verträgliche Salze in Frage. Hierzu gehören Salze dieser Verbindungen mit anorganischen oder organischen Säuren, vorzugsweise Halogenwasserstoffsäuren, wie Salzsäure und Bromwasserstoffsäure ; Salpetersäure ; Phosphorsäure ; Schwefelsäure ; mono- und bifunktionellen Carbonsäuren und Hydroxycarbonsäuren, wie Essigsäure, Oxalsäure, Maleinsäure, Bernsteinsäure, Fumarsäure, Weinsäure, Zitronensäure, Salicylsäure, Sorbinsäure und Milchsäure ; und Sulfonsäuren, wie 1,5-Naphthalin-disulfonsäure.

Eine interessante Untergruppe von Verbindungen der Formel I besteht aus denjenigen Verbindungen der Formel I, in denen $R^1$ die obige Bedeutung besitzt, $R^2$ mit 1 bis 3 Fluor-, Chlor-, Brom- und/oder Jodatomen, 1 oder 2 $C_{1-4}$-Alkylgruppen, 1 oder 2 Trifluormethylgruppen, 1 oder 2 $C_{1-2}$-Alkoxygruppen, einer Methylthiogruppe, einer Cyanogruppe und/oder einer Nitrogruppe substituiertes Phenyl oder gegebenenfalls mit 1 bis 3 Chloratomen und/oder einer Methylgruppe substituiertes 2-, 3- oder 4-Pyridyl bedeutet und $R^3$ o-Trifluormethyl-phenyl bedeutet.

Unabhängig voneinander bedeuten $R^1$ vorzugsweise $C_{1-4}$-Alkyl, insbesondere Methyl oder Aethyl, und $R^2$ vorzugsweise substituiertes Phenyl, das zumindest einen Substituenten in einer ortho-Stellung aufweist, insbesondere ein ortho Halogenatom. $R^2$ ist speziell bevorzugt o-Halogenphenyl, o,o'-Dihalogenphenyl oder o,p-Dihalogenphenyl.

Besonders bevorzugte Verbindungen der Formel I sind :

3-(o-Chlorphenyl)-1-methyl-5-(o-trifluormethyl-phenyl)-1H-1,2,4-triazol,

3-(o,o'-Difluorphenyl)-1-methyl -5-(o-trifluormethyl-phenyl)-1H-1,2, 4-triazol,

3-(o-Bromphenyl)-1-methyl-5-(o-trifluormethyl-phenyl)-1H-1,2,4-triazol,

3-(2-Chlor-6-fluorphenyl)-1-methyl-5-(o-trifluormethyl-phenyl)-1H-1,2,4-triazol,
3-(o,p-Dichlorphenyl)-1-methyl-5-(o-trifluormethyl-phenyl)-1H-1,2,4-triazol und
3-(2-Chlor-4-fluorphenyl)-1-methyl-5-(o-trifluormethyl-phenyl)-1H-1,2,4-triazol.

Weitere bevorzugte Verbindungen der Formel I sind :
3-(o-Jodphenyl)-1-methyl-5-(o-trifluormethyl-phenyl)-1H-1,2,4-triazol,
3-(o,p-Difluorphenyl)-1-methyl-5-(o-trifluormethyl-phenyl)-1H-1,2,4-triazol,
3-(o-Chlorphenyl)-1-methyl-5-(4-trifluormethyl-3-pyridyl)-1H-1,2,4-triazol,
3-(o-Fluorphenyl)-1-methyl-5-(o-trifluormethyl-phenyl)-1H-1,2,4-triazol,
1-Aethyl-3-(o-chlorphenyl)-5-(o-trifluormethyl-phenyl)-1H-1,2,4-triazol,
1-Aethyl-3-(o,o'-difluorphenyl)-5-(o-trifluormethyl-phenyl)-1H-1,2,4-triazol und
3-(o-Cyanophenyl)-1-methyl-5-(o-trifluormethyl-phenyl)-1H-1,2,4-triazol.

Weitere Vertreter von Verbindungen der Formel I sind :
3-(2-Chlor-4-nitrophenyl)-1-methyl-5-(o-trifluormethyl-phenyl)-1H-1,2,4-triazol,
3-(2-Chlor-3-pyridyl)-1-methyl-5-(o-trifluormethyl-phenyl)-1H-1,2,4-triazol,
3-(o-Methylthiophenyl)-1-methyl-5-(o-trifluormethyl-phenyl)-1H-1,2,4-triazol,
3-(2-Chlor-4-trifluormethyl-phenyl)-1-methyl-5-(o-trifluormethyl-phenyl)-1H-1,2,4-triazol,
1-Methyl-3-(2,3,6-trichlorphenyl)-5-(o-trifluormethyl-phenyl)-1H-1,2,4-triazol,
1-Methyl-3-(p-nitrophenyl)-5-(o-trifluormethyl-phenyl)-1H-1,2,4-triazol,
1-Methyl-3-(o-nitrophenyl)-5-(o-trifluormethyl-phenyl)-1H-1,2,4-triazol,
3-(2-Chlor-4-pyridyl)-1-methyl-5-(o-trifluormethyl-phenyl)-1H-1,2,4-triazol,
3-(4-Chlor-3-pyridyl)-1-methyl-5-(o-trifluormethyl-phenyl)-1H-1,2,4-triazol,
1-Methyl-3-(2,4,6-trifluorphenyl)-5-(o-trifluormethyl-phenyl)-1H-1,2,4-triazol,
1-Methyl-3-pentafluorphenyl-5-(o-trifluormethyl-phenyl)-1H-1,2,4-triazol,
3-(o-Aethylphenyl)-1-methyl-5-(o-trifluormethyl-phenyl)-1H-1,2,4-triazol,
3-(2-Chlor-6-methylphenyl)-1-methyl-5-(o-trifluormethyl-phenyl)-1H-1,2,4-triazol,
3-(o,p-Dimethylphenyl)-1-methyl-5-(o-trifluormethyl-phenyl)-1H-1,2,4-triazol,
3-(2-Chlor-4-methylphenyl)-1-methyl-5-(o-trifluormethyl-phenyl)-1H-1,2,4-triazol,
3-(4-Chlor-2-methylphenyl)-1-methyl-5-(o-trifluormethyl-phenyl)-1H-1,2,4-triazol,
3-(o-Trifluormethoxy-phenyl)-1-methyl-5-(o-trifluormethyl-phenyl)-1H-1,2,4-triazol,
3-(o-Difluormethyl-phenyl)-1-methyl-5-(o-trifluormethyl-phenyl)-1H-1,2,4-triazol,
3-(o-Chlordifluormethyl-phenyl)-1-methyl-5-(o-trifluormethyl-phenyl)-1H-1,2,4-triazol,
3-(o-Dichlormethyl-phenyl)-1-methyl-5-(o-trifluormethyl-phenyl)-1H-1,2,4-triazol,
1-Chlormethyl-3-(o-chlorphenyl)-5-(o-trifluormethyl-phenyl)-1H-1,2,4-triazol,
3-(o-Chlorphenyl)-5-(o-trifluormethyl-phenyl)-1-vinyl-1H-1,2,4-triazol,
3-(o,o'-Difluorphenyl)-1-methyl-5-(4-trifluormethyl-3-pyridyl)-1H-1,2,4-triazol und
3-(2-Chlor-4-fluorphenyl)-1-methyl-5-(4-trifluormethyl-3-pyridyl)-1H-1,2,4-triazol.

Das erfindungsgemässe Verfahren zur Herstellung der Verbindungen der Formel I und von deren Säureadditionssalzen ist dadurch gekennzeichnet, dass man

a) ein 1,2,4-Triazol der allgemeinen Formel

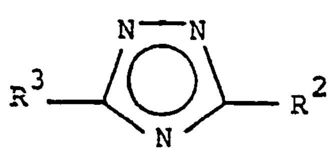

II

worin $R^2$ und $R^3$ die oben angegebenen Bedeutungen besitzen,
mit einer Verbindung der allgemeinen Formel

$$R^1 - X \qquad III$$

worin
$R^1$ die oben angegebene Bedeutung besitzt
und X eine Abgangsgruppe, wie Halogen (insbesondere Chlor, Brom oder Jod), Mesyloxy oder Tosyloxy, bedeutet, umsetzt,

b) eine Verbindung der allgemeinen Formel

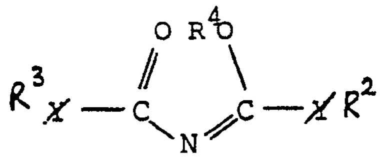 IV

worin $R^2$ und $R^3$ die oben angegebenen Bedeutungen besitzen, und $R^4$ einen Niederalkylrest bedeutet, oder ein Säureadditionssalz davon mit einem Hydrazin der allgemeinen Formel

$$R^1\text{-NH-NH}_2 \qquad \text{V}$$

worin $R^1$ die oben angegebene Bedeutung besitzt, umsetzt,

c) zwecks Herstellung derjenigen Verbindungen der Formel I, in denen $R^1$ $C_{1\text{-}4}$-Alkyl oder $C_{2\text{-}4}$-Alkenyl bedeutet, einen Imidsäureester der allgemeinen Formel

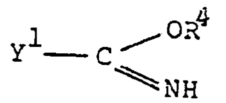 VI

worin $Y^1$ für $R^3$ oder für $R^2$ steht und $R^4$ und $R^2$ bzw. $R^3$ die oben angegebenen Bedeutungen besitzen, oder ein Säureadditionssalz davon, mit einem Säurehydrazid der allgemeinen Formel

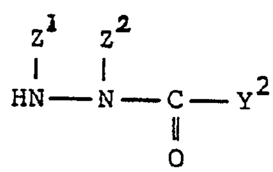 VII

worin $Y^2$ für $R^2$, $Z^1$ für $C_{1\text{-}4}$-Alkyl oder $C_{2\text{-}4}$-Alkenyl und $Z^2$ für Wasserstoff stehen (falls $Y^1$ der Formel VI $R^3$ bedeutet) oder $Y^2$ für $R^3$, $Z^1$ für Wasserstoff und $Z^2$ für $C_{1\text{-}4}$-Alkyl oder $C_{2\text{-}4}$-Alkenyl stehen (falls $Y^1$ der Formel VI $R^2$ bedeutet), wobei $R^2$ bzw. $R^3$ die oben angegebene Bedeutung besitzt,

zu einem Acylamidrazon umsetzt und dieses durch Erhitzen cyclisiert,

d) zwecks Herstellung derjenigen Verbindungen der Formel I, in denen $R^1$ $C_{1\text{-}4}$-Alkyl oder $C_{2\text{-}4}$-Alkenyl bedeutet, ein Diacyl-, Monothiodiacyl- bzw. Dithiodiacylamin der allgemeinen Formel

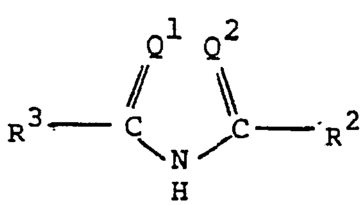 VIII

worin $R^2$ und $R^3$ die oben angegebenen Bedeutungen besitzen und $Q^1$ und $Q^2$ unabhängig voneinander Sauerstoff oder Schwefel bedeuten,

mit einem Hydrazin der allgemeinen Formel

$$R^{1'}\text{-NH-NH}_2 \qquad \text{V'}$$

worin $R^1$ $C_{1-4}$-Alkyl oder $C_{2-4}$-Alkenyl bedeutet, umsetzt, oder
e) zwecks Herstellung derjenigen Verbindungen der Formel I, in denen $R^1$ $C_{1-4}$-Halogenalkyl bedeutet, ein 1-Hydroxyalkyl1H-1,2,4-triazol der allgemeinen Formel

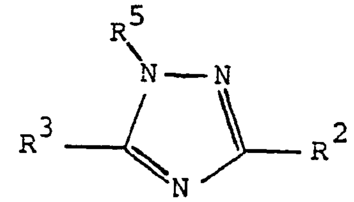

IX

worin $R^5$ $C_{1-4}$-Hydroxyalkyl bedeutet. und $R^2$ und $R^3$ die oben angegebenen Bedeutungen besitzen, mit einem Halogenierungsmittel behandelt, und erwünschtenfalls eine so erhaltene Verbindung der Formel I durch Umsetzung mit einer Säure in das entsprechende Säureadditionssalz überführt.

Die Umsetzung nach Verfahrensvariante a) wird zweckmässigerweise in einem inerten Verdünnungsmittel sowie in Gegenwart einer Base durchgeführt. Als Verdünnungsmittel eignen sich insbesondere inerte organische Lösungsmittel, wie niedere Alkanole, z.B. Methanol oder Aethanol, aliphatische oder cyclische Aether, z.B. Diäthyläther oder Tetrahydrofuran, und dipolare aprotische Lösungsmittel, z.B. Dimethylformamid. Vorzugsweise wird ein Alkali-Alkoholat, z.B. Natriummethylat oder Natriumäthylat, oder Natriumhydrid als Base benützt. Die Reaktionstemperaturen können bei der Durchführung dieser Verfahrensvariante in einem grossen Bereich variiert werden, wobei im allgemeinen bei Temperaturen zwischen –20°C und 70°C bzw. vorzugsweise zwischen 0°C und 30°C gearbeitet wird.

Da die Formel II die Verbindungen der allgemeinen Formeln

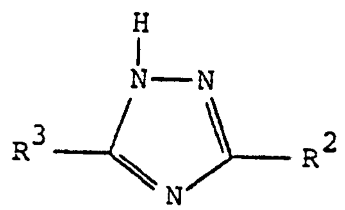

und

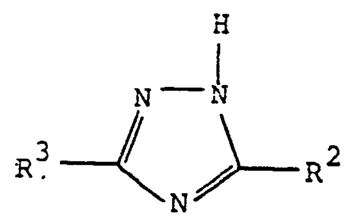

IIa

IIb

umfasst, fällt das Produkt der Formel I normalerweise als Gemisch mit dem entpsprechenden 1-$R^1$-3-$R^3$-5-$R^2$-1H-1,2,4-triazol an. Aus dem Gemisch kann die Verbindung der Formel nach an sich bekannten Trennungsmethoden, z.B. durch Säulenchromatographie, isoliert werden.

Im Falle der Verfahrensvarianten b) und c) ist unter "Niederalkylrest" ($R^4$) insbesondere ein 1 bis 6 Kohlenstoffatome enthaltender Alkylrest zu verstehen, vorzugsweise jedoch Methyl oder Aethyl. Beispiele von Säureadditionssalzen der Verbindungen der Formel IV bzw. VI sind Hydrochlorid und Hydrobromid.

Die Umsetzung nach Verfahrensvariante b) erfolgt zweckmässigerweise in einem inerten Verdünnungsmittel, insbesondere einem chlorierten Kohlenwasserstoff, z.B. Methylenchlorid oder Tetrachlorkohlenstoff, einem aliphatischen oder cyclischen Aether, z.B. Tetrahydrofuran, oder einem aliphatischen Nitril, z.B. Acetonitril. Auch bei der Durchführung dieser Verfahrensvariante können die Reaktionstemperaturen in einem grossen Bereich variiert werden, wobei vorzugsweise bei Temperaturen zwischen 10°C und 50°C gearbeitet wird.

Die Umsetzung nach Verfahrensvariante c) kann je nach Reaktionsteilnehmern mit oder ohne Verwendung eines inerten Verdünnungsmittels durchgeführt werden. Falls der eine oder die beiden Reaktionsteilnehmer nicht ohne Zersetzung geschmolzen werden können, erfolgt die Umsetzung zweckmässigerweise in einem inerten organischen Lösungsmittel, wie einem aliphatischen Alkohol, z.B. Methanol ; einem Aromaten,z.B. Toluol oder einem Xylol ; einem halogenierten aromatischen Kohlenwasserstoff, z.B. Chlorbenzol ; oder Dimethylformamid. Das Reaktionsgemisch wird bei erhöhter Temperatur gehalten, und zwar vorteilhaft im Temperaturbereich von ca. 80°C bis ca. 150°C. Im Falle der Verwendung eines Verdünnungsmittels wird geeigneterweise zunächst auf die Rückflusstemperatur des Reaktionsgemisches erhitzt und nach Abdampfen des Verdünnungsmittels das Erhitzen gegebenenfalls bei höheren Temperaturen. z.B. bis

auf ca. 200°C, fortgesetzt, um die Cyclisierung des als Zwischenprodukt gebildeten Acylamidrazons der allgemeinen Formel

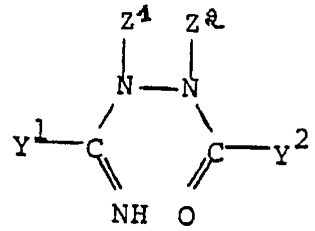

X

worin $Y^1$, $Y^2$, $Z^1$ und $Z^2$ die oben angegebenen Bedeutungen besitzen, zu beschleunigen. Zum selben Zweck und unabhängig von der An- oder Abwesenheit eines Verdünnungsmittels kann man einen sauren Katalysator einsetzen, wie eine Mineralsäure, z.B. Salzsäure oder Bromwasserstoffsäure, oder eine aromatische Sulfonsäure, z.B. p-Toluolsulfonsäure, oder ein Säureadditionssalz, z.B. das Hydrochlorid des Imidesters der Formel VI.

Die Verfahrensvariante d) stellt eine Einhorn-Brunner-Reaktion dar ; diese erfolgt zweckmässigerweise unter Verwendung eines Verdünnungsmittels, insbesondere eines organischen Lösungsmittels, wie eines Alkohols, z.B. Aethanol, eines aliphatischen oder cyclischen Aethers, z.B. Dioxan, eines chlorierten Aromaten, z.B. Chlorbenzol, oder Essig Essigsäure, bei erhöhter Temperatur, vorzugsweise bei Temperaturen zwischen ca. 50°C und der Rückflusstemperatur des Reaktionsgemisches.

Die Halogenierung gemäss der Verfahrensvariante e) kann im allgemeinen nach dem Fachmann geläufigen Methoden durchgeführt werden. Es wird beispielsweise Phosphorpentachlorid, Thionylchlorid oder Phosphoroxychlorid als Chlorierungsmittel bzw. Phosphortribromid als Bromierungsmittel verwendet. Bei der Durchführung wird zweckmässigerweise in Gegenwart eines inerten Verdünnungsmittels, insbesondere eines aprotischen organischen Lösungsmittels, und gegebenenfalls auch in Gegenwart einer Base gearbeitet. Zu den bevorzugten Verdünnungsmitteln gehören aliphatische und aromatische Kohlenwasserstoffe, wie n-Hexan, Benzol, Toluol und Xylole ; halogenierte aliphatische Kohlenwasserstoffe, wie Methylenchlorid, Chloroform und Tetrachlorkohlenstoff ; halogenierte aromatische Kohlenwasserstoffe, wie Chlorbenzol ; und tertiäre Amine, wie Triäthylamin und Pyridin. Bevorzugte Basen sind Triäthylamin, Pyridin und Calciumcarbonat. Die Reaktionstemperaturen liegen im allgemeinen zwischen 0°C und der Rückflusstemperatur des Reaktionsgemisches, vorzugsweise zwischen der Raumtemperatur und der Rückflusstemperatur. Das Halogenierungsmittel wird vorzugsweise im Ueberschuss verwendet.

Zur Herstellung der Säureadditionssalze der Verbindungen der Formel I werden die Verbindungen I mit den gewünschten Säuren auf übliche Weise umgesetzt, beispielsweise durch Lösen der Verbindung der Formel I in einem geeigneten Lösungsmittel, wie Diäthyläther, Aethanol, Aethylacetat, Toluol oder Methylenchlorid, und Hinzufügen der Säure, wie Chlorwasserstoff in Form von konzentrierter oder gasförmiger Salzsäure. Der resultierende Niederschlag des Säureadditionssalzes kann anschliessend z.B. durch Filtrieren abgetrennt werden.

Die Isolierung und die Reinigung der so hergestellten Verbindungen der Formel I bzw. der Säureadditionssalze können nach an sich bekannten Methoden durchgeführt werden. Die Verbindung der Formel I kann beispielsweise in Form ihres Säureadditionssalzes isoliert und dies mit Natronlauge zur Freisetzung des freien 1,2,4-Triazols versetzt werden, das seinerseits durch Umkristallisieren, Destillieren oder Säulenchromatographie gereinigt wird.

Die als Ausgangsmaterialien in der Verfahrensvariante a) verwendeten 1,2,4-Triazole der Formel II sind neu und können beispielsweise aus Azinen der allgemeinen Formel

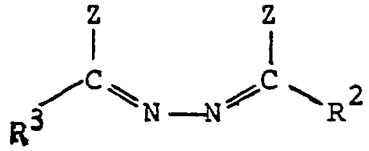

XI

worin Z eine Abgangsgruppe, wie Halogen, vorzugsweise Chlor, bedeutet un $R^2$ und $R^3$ die oben angegebenen Bedeutungen besitzen, hergestellt werden, und zwar i) durch Umsetzung mit Ammoniak, zweckmässigerweise in einem organischen Lösungsmittel, wie einem niederen Alkanol, z.B. Aethanol, einem

aliphatischen oder cyclischen Aether, wie Tetrahydrofuran, oder einem Aromaten, z.B. Toluol, bei erhöhter Temperatur, wie im Temperaturbereich von 30°C bis 100°C, vorzugsweise jedoch bei der Rückflusstemperatur des Reaktionsgemisches, oder ii) durch Umsetzung mit Hydrazin und anschliessende Säurebehandlung und reduktive Desaminierung. Die Umsetzung mit Hydrazin erfolgt zweckmässigerweise in einem inerten Verdünnungsmittel, wie einem im Zusammenhang mit i) (Umsetzung mit Ammoniak) angegebenen, bei erhöhter Temperatur, wie im Temperaturbereich von 20°C bis 100°C, vorzugsweise 40°C bis 80°C. Die anschliessende Säurebehandlung wird zweckmässigerweise unter Verwendung einer starken Mineralsäure, wie konzentrierter Salzsäure, mit Erhitzen bewerkstelligt, wonach neu tral gestellt wird. Das als Zwischenprodukt gebildete 4-Amino-1,2,4-triazol der allgemeinen Formel

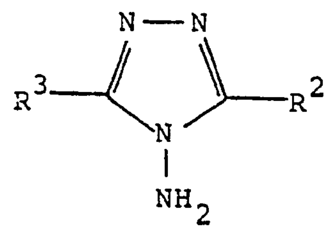

XII

wird dann zweckmässigerweise durch Behandlung mit salpetriger Säure desaminiert, wobei diese Säure geeigneterweise in situ aus einem Alkalimetallnitrit, wie Natriumnitrit, in Gegenwart einer Säure, wie Essigsäure, erzeugt wird. Die Desaminierung erfolgt bei tiefen Temperaturen, insbesondere zwischen 0°C und 20°C.

Die Azine der Formel XI sind ihrerseits aus den entsprechenden Diacylhydrazinen der allgemeinen Formel

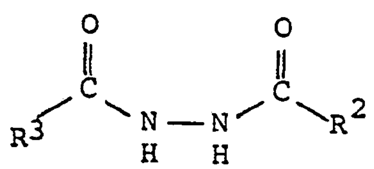

XIII

herstellbar, z.B. durch Chlorierung mit Phosphorpentachlorid bei erhöhter Temperatur, zweckmässigerweise in einem chlorierten Aromaten, wie dies beispielsweise in Liebigs Annalen der Chemie 749, 5 und 11 (1971) beschrieben ist.

Die 1,2,4-Triazole der Formel II können zudem analog den Verfahrensvarianten b), c) und d) hergestellt werden. wobei anstelle des Hydrazins der Formel V ein unsubstituiertes Hydrazin bzw. anstelle des Säurehydrazids der Formel VII ein Säurehydrazid der Formel

$$H_2NNHCOY^2 \qquad \text{XIV}$$

worin $Y^2$ die oben angegebene Bedeutung besitzt, eingesetzt wird.

Die Ausgangsmaterialien der Formeln III und V bzw. V' sind entweder bekannt oder können nach an sich bekannten Methoden hergestellt werden. Ebenfalls zum grössten Teil bekannt sind die als Ausgangsmaterialien in der Verfahrensvariante b) verwendeten Verbindungen der Formel IV ; diese lassen sich insbesondere in an sich bekannter Weise durch Acylierung der Imidester der Formel VI mit einem die Gruppe der Formel YCO- enthaltenden Acylierungsmittel, wie dem entsprechenden Säurechlorid, -bromid oder -anhydrid, in Gegenwart eines organischen oder anorganischen Säureacceptors, z.B. Natriumcarbonat, Triäthylamine oder Pyridin, herstellen.

Die als Ausgangsmaterialien in der Verfahrensvariante c) und zur Herstellung der Verbindungen der Formel IV verwendeten Imidsäureester der Formel VI und deren Säureadditionssalze sind entweder bekannt oder können in an sich bekannter Weise hergestellt werden, beispielsweise durch Behandlung eines Nitrils $Y^1CN$ mit einem wasserfreien Alkohol $R^4OH$ in Gegenwart eines sauren Katalysators, wie Chlorwasserstoff (Pinner-Reaktion), oder durch O-Alkylierung eines Amids $Y^1CONH_2$ mit einem den Rest $R^4$ ent-

haltenden Alkylierungsmittel, wie einem Meerwein-Salz, z.B. Triäthyloxonium-tetrafluorborat, wie dies in J. Org. Chem. 33, 1679-1680 (1968) beschrieben ist. Die auf diese Weise hergestellten Säureadditionssalze der Imidsäureester VI können durch konventionelle Behandlung mit Basen in die entsprechenden freien Basen übergeführt werden.

Die als Ausgangsmaterialien in der Verfahrensvariante c) verwendeten Säurehydrazide der Formel VII sowie die Säurehydrazide hydrazide der Formel XIV sind entweder bekannt oder können nach an sich bekannten Methoden hergestellt werden, beispielsweise durch Acylierung von Hydrazin bzw. $C_{1-4}$-Monoalkylhydrazinen mit die Gruppe der Formel YCO- enthaltenden Carbonsäureestern oder -anhydriden. wie dies u.a. in J.A.C.S. 80, 1895-1900 (1958) und J. Chem. Soc. (B) 1969, 1185-1191, beschrieben ist.

Auch die als Ausgangsmaterialien in der Verfahrensvariante d) verwendeten Diacylamine, Monothiodiacylaminne bzw. Dithiodiacylamine der Formel VIII sind bekannt oder nach an sich bekannten Methoden herstellbar, und zwar beispielsweise durch Acylierung von Amiden oder Thioamiden $R^3$-$CQ^1NH_2$ mit Acylhalogeniden oder Thioacylhalogeniden $R^2$-$CQ^2$-Hal, worin $R^2$, $R^3$, $Q^1$ und $Q^2$ die oben angegebenen Bedeutungen besitzen und Hal Halogen, insbesondere Chlor oder Brom, bedeutet. Solche Herstellungsmethoden sind u.a. in Rocz. Chem. 48, 243 (1974), J. Het. Chem. 19, 613 (1982), J. Het. Chem. 20, 1693-1695 (1983) und US-Patentschrift Nr. 4.414.221 beschrieben.

Die als Ausgangsmaterialien in der Verfahrensvariante e) eingesetzten 1-Hydroxyalkyl-1H-1,2,4-triazole der Formel IX sind neu ; sie können beispielsweise analog der Verfahrensvariante a) oder b) hergestellt werden, wenn dort anstelle der Verbindung der Formel III oder V eine Verbindung der Formel $R^5$-X bzw. $R^5$-$NH$-$NH_2$, worin $R^5$ $C_{1-4}$-Hydroxyalkyl bedeutet, eingesetzt wird.

Die Isolierung und die Reinigung der so hergestellten Ausgangsmaterialien können nach an sich bekannten Methoden durchgeführt werden.

Die erfindungsgemässen Verbindungen, d.h. die Verbindungen der Formel I und ihre Säureadditionssalze,sind ganz allgemein als Pestizide von Wert. Sie erweisen sich als besonders wertvoll zur Bekämpfung von Insekten und Milben, insbesondere von

– Homoptera (insbesondere Blattläusen), wie z.B. Aphis fabae, Aphis gossypii, Aphis pomi ; Acyrthosiphon pisum, Acyrthosiphon dirhodum ; Brevicoryne brassicae ; Dysaphis devecta, Dysaphis pyri, Dysaphis plantaginea ; Macrosiphum rosae, Macrosiphum avenae ; Myzus persicae, Myzus cerasi ; Phorodon humuli ; Rhopalosiphum insertum, Rhopalosiphum padi ; Toxoptera aurantii ; Nasonovia ribisnigri ; Hyalopterus pruni ;
– Weissen Fliegen, wie z.B. Trialeurodes vaporariorum ; Aleurothrixus floccosus ; Bemisia tabaci ; Aleurodes proletella ; Aleurocanthus woglumi ; Dialeurodes citri ;
– Blattsaugern, wie z.B. Psylla mali, Psylla piri, Psylla pirisuga, Psylla piricula ; Trioza apicalis ;
– Milben, die bei dem Pflanzenschutz von Bedeutung sind, wie z.B. Tetranychidae (Spinnmilben), insbesondere Tetranychus urticae, Tetranychus cinnabarinus, Tetranychus turkestani, Tetranychus McDanieli, Tetranychus kanzawai ; Panonychus ulmi, Panonychus citri ; Phyllocoptruta oleivora ; Aculus schlechtendali ; Phyllocoptes vitis ; Aceria essigi, Aceria gracilis ; Cecidophyopsis ribis ; Eriophyes vitis ; Eotetranychus sexmaculatus, Eotetranychus carpini ; Hemitarsonemus latus ;
– Milben, die in der Veterinärmedizin von Bedeutung sind, wie z.B. Macronyssus bursa, Macronyssus sylviarum, Macronyssus lacoti ; Dermanyssus gallinae ; Zecken, insbesondere der Familien Ixodidae und Argasidae und der Ordnungen Boophilus, Amblyomma, Hyalomma, Rhipicephalus, Ixodes, Argas und Ornithodorus.

Die erfindungsgemässen Verbindungen wirken als Kontakt- und Frassgifte. Zudem werden einige der Verbindungen von verschiedenen Pflanzen aufgenommen, so dass die zu bekämpfenden Schädlinge beim Fressen der Pflanzen vernichtet werden. Diese Verbindungen weisen also systemische Wirkung auf.

Das erfindungsgemässe Schädlingsbekämpfungsmittel ist dadurch gekennzeichnet, dass es eine wirksame Menge mindestens einer Verbindung der allgemeinen Formel I, wie oben definiert, oder eines Säureadditionssalzes einer solchen Verbindung sowie Formulierungshilfsstoffe enthält. Das Mittel enthält zweckmässigerweise zumindest einen der folgenden Formulierungshilfsstoffe :

Feste Trägerstoffe ; Lösungs- bzw. Dispersionsmittel ; Tenside (Netz- und Emulgiermittel) ; Dispergatoren (ohne Tensidwirkung) ; und Stabilisatoren.

Unter Verwendung solcher und anderer Hilfsstoffe können die Verbindungen der Formel I, also die pestiziden Wirkstoffe, in die üblichen Formulierungen übergeführt werden, wie Lösungen, Suspensionen, Emulsionen, emulgierbare Konzentrate, Pasten, Schäume, Stäube, Pulver und Granulate.

Als feste Trägerstoffe kommen im wesentlichen in Frage : natürliche Mineralstoffe, wie Kaolin, Tonerden, Kieselgur, Talkum, Bentonit, Kreide, Kalkstein, Quarz, Dolomit, Attapulgit, Montmorillonit und Diatomeenerde ; synthetische Mineralstoffe, wie hochdisperse Kieselsäure, Aluminiumoxid und Silikate ; organische Stoffe, wie Cellulose, Stärke, Harnstoff und Kunstharze ; und Düngemittel, wie Phosphate und

Nitrate, wobei solche Trägerstoffe z.B. als Stäube, Pulver oder Granulate vorliegen können.

Als Lösungs- bzw. Dispersionsmittel kommen im wesentlichen in Frage : Aromaten, wie Toluol, Xylole, Benzol und Alkylnaphthaline ; chlorierte Aromaten und chlorierte aliphatische Kohlenwasserstoffe, wie Chlorbenzole, Chloräthylene und Methylenchlorid ; aliphatische Kohlenwasserstoffe, wie Cyclohexan und Paraffine, z.B. Erdölfraktionen ; Alkohole, wie Butanol und Glykol, sowie deren Aether und Ester ; Ketone, wie Aceton, Methyläthylketon, Methylisobutylketon und Cyclohexanon ; und stark polare Lösungsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz- und Emulgiermittel) können nicht-ionische Verbindungen sein, wie Kondensationsprodukte von Fettsäuren, Fettalkoholen oder fettsubstituierten Phenolen mit Aethylenoxid ; Fettsäureester und -äther von Zuckern oder mehrwertigen Alkoholen ; die Produkte, die aus Zuckern oder mehrwertigen Alkoholen durch Kondensation mit Aethylenoxid erhalten werden ; Blockpolymere von Aethylenoxid und Propylenoxid ; oder Alkyldimethylaminoxide.

Die Tenside können auch anionische Verbindungen darstellen, wie Seifen ; Fettsulfatester, z.B. Dodecylnatriumsulfat, Octadecylnatriumsulfat und Cetylnatriumsulfat ; Alkylsulfonate, Arylsulfonate und fettaromatische Sulfonate, wie Alkylbenzolsulfonate, z.B. Calcium-Dodecylbenzolsulfonat, und Butylnaphthalinsulfonate ; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage : Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide ; Antioxidantien, z.B. Gallussäureester und Butylhydroxytoluol ; UV-Absorber, z.B. substituierte Benzophenone, Diphenylacrylnitrilsäureester und Zimtsäureester ; und Deaktivatoren, z.B. Salze der Aethylendiaminotetraessigsäure und Polyglykole.

Die erfindungsgemässen Schädlingsbekämpfungsmittel können neben den Wirkstoffen der Formel I auch andere Wirkstoffe enthalten, z.B. anderweitige Schädlingsbekämpfungsmittel, Schädlingslockstoffe, Fungizide, Bakterizide, Pflanzenwachstumsregulatoren und Düngemittel. Solche Kombinationsmittel eignen sich zur Verstärkung der Aktivität bzw. zur Verbreiterung des Wirkungsspektrums. Gegebenenfalls können dadurch auch Unzulänglichkeiten von bisher bekannten zugesetzten Mitteln ausgeglichen werden.

Es wurde gefunden, dass die erfindungsgemässen Verbindungen, insbesondere die als besonders bevorzugt hervorgehobenen, mit Vorteil in Kombination mit anderen Akariziden, vor allem mit zur Bekämpfung von mobilen Stadien von Milben geeigneten, eingesetzt werden. Beispiele derartiger Akarizide sind Amitraz, Avermectin, Benzoximate, Bromopropylate, Chlorobenzilate, Cyhexatin, Dicofol, Fenbutatin oxide, Methidathion, Propargite und Aethyl-5,7-dihydro-6H-dibenz[c,e]azepin-6-carboximidat sowie Pyrethroide mit akarizider Wirkung, wie beispielsweise Fluvalinate, Biphenthrin und Cyano-3-phenoxybenzyl-3-(2-chlor-2,3,3-trifluorprop-1-enyl)-2,2-dimethyl-cyclopropancarboxylat. Die Anwendung kann gleichzeitig als Mischung oder getrennt erfolgen. Dabei können die erfindungsgemässen Wirkstoffe den Nachteil der bekannten Akarizide mit Wirkungsschwerpunkt gegen adulte Schädlinge egalisieren, indem die nach Einsatz der bekannten Akarzide überlebenden Eier und Larven, welche sich rasch zu einer neuen schädlichen Population entwickeln können, ebenfalls abgetötet werden.

Die erfindungsgemässen Schädlingsbekämpfungsmittel enthalten im allgemeinen zwischen 0,005 und 95 Gewichtsprozent einer erfindungsgemässen Verbindung bzw. von erfindungsgemässen Verbindungen als Wirkstoff(e). Sie können in einer Form vorliegen, die sich für die Lagerung und den Transport eignet. In solchen Formen, z.B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zur Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im allgemeinen 5 bis 90 Gewichtsprozent, vorzugsweise 10 bis 80 Gewichtsprozent, der erfindungsgemässen Verbindungen. Als Anwendungsformen kommen u.a. gebrauchsfertige Lösungen, Emulsionen, Suspensionen, Schäume, Pulver, Pasten, Stäubemittel und Granulate in Frage. Die Wirkstoffkonzentrationen in solchen anwendungsfertigen Zubereitungen können in grossen Bereichen variiert werden. In Spritzbrühen können z.B. Konzentrationen zwischen 0,005 und 0,5 Gewichtsprozent vorliegen. Im Ultra-Low-Volume-Verfahren können Spritzbrühen formuliert werden, in denen die Wirkstoffkonzentration vorzugsweise von 10 bis 20 Gewichsprozent beträgt, während die im Low-Volume-Verfahren und im High-Volume-Verfahren formulierten Spritzbrühen vorzugsweise eine Wirkstoffkonzentration von 0,01 bis 0,5 bzw. 0,005 bis 0,1 Gewichtsprozent aufweisen. Granulate enthalten vorzugsweise von 5 bis 50 Gewichtsprozent der erfindungsgemässen Verbindung(en) als Wirkstoff(e).

Die erfindungsgemässen Schädlingsbekämpfungsmittel können dadurch hergestellt werden, dass man mindestens eine Verbindung der allgemeinen Formel I bzw. ein Säureadditionssalz einer solchen Verbindung mit Formulierungshilfsstoffen vermischt.

Die Herstellung der Mittel kann in bekannter Weise durchgeführt werden, z.B. durch Vermischen des Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln.

Im Falle von pulverförmigen Mitteln kann der Wirkstoff mit einem festen Trägerstoff vermischt werden, z.B. durch Zusammenmahlen ; oder man kann den festen Trägerstoff mit einer Lösung oder Suspension des Wirkstoffes imprägnieren und dann das Lösungs- oder Suspensionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindungen der Formel I bzw. deren Säureadditionssalze können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines Granulates vermischt werden.

Wenn gewünscht, kann die Verbindung der Formel I oder ein Säureadditionssalz davon in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Das erfindungsgemässe Verfahren zur Bekämpfung von Schädlingen ist dadurch gekennzeichnet, dass man das zu schützende Gut oder die Schädlinge selbst mit einer wirksamen Menge einer erfindungsgemässen Verbindung bzw. eines erfindungsgemässen Schädlingsbekämpfungsmittels behandelt. Dieses Anwendungsverfahren kann durch Boden- oder Blattapplikation bzw. durch Applikation auf die zu schützenden Tiere, Vorräte oder Materialien, je nach Art der zu bekämpfenden Schädlinge, durchgeführt werden. Die Bekämpfung wird beispielsweise durch Kontakt oder durch Einnahme mit der Nahrung erzielt.

Die Anwendung kann in konventioneller Weise geschehen, z.B. durch Verspritzen, Versprühen, Vernebeln, Verstäuben, Verstreuen, Eindrillen, Verräuchern, Giessen, Beizen oder Inkrustieren. Pulverförmige Präparate können z.B. als Stäubemittel mit Hilfe der üblichen Verstäubegeräte auf die Schädlinge bzw. auf das zu schützende Gut, z.B. Pflanzen oder Tiere, aufgebracht werden. Wässrige Suspensionen sind z.B. als Spritzmittel anwendbar.

Bei der Anwendung im Pflanzenschutz genügt in der Regel eine Dosierung von ca. 120-500 g Wirkstoff [Verbindung(en) der Formel I]/ha, z.B. wie dies bei der Applikation von 2000 l einer Spritzbrühe, die 0,006-0,025 Gewichtsprozent Wirkstoff enthält, auf 1 ha bepflanzten Erdbodens der Fall ist.

Die nachfolgenden Beispiele dienen zur näheren Erläuterung der Erfindung.

I. Herstellung der Wirkstoffe der Formel I :

Beispiel 1

0,19 g Natriumhydrid werden in 10 ml N,N-Dimethylformamid vorgelegt. Unter Rühren lässt man eine Lösung von 2,3 g 3-(o-Fluorphenyl)-5-(o-trifluormethyl-phenyl)-1H-1,2,4-triazol in 15 ml N,N-Dimethylformamid während 10 Minuten zutropfen. Nach 45 Minuten Nachrühren versetzt man die inzwischen braun gewordene Lösung unter Eiskühlung tropfenweise mit 1,17 g Methyljodid. Das Reaktionsgemisch wird eine Stunde nachgerührt und danach auf Eiswasser gegossen, und das wässrige Gemisch wird dreimal mit Aethylacetat extrahiert. Die vereinigten Extrakte werden mit Wasser und gesättigter Natriumchloridlösung gewaschen, über wasserfreiem Natriumsulfat getrocknet und unter vermindertem Druck eingedampft. Auf diese Weise erhält man ein Gemisch der isomeren N-Methyltriazole, welche sich durch Chromatographie an Kieselgel auftrennen lassen. Durch Eluieren mit n-Hexan/Aethylacetat (19 : 1) erhält man reines 5-(o-Fluorphenyl)-1-methyl-3-(o-trifluormethyl-phenyl)-1H-1,2,4-triazol als harzartiges Produkt, $^1$H-NMR-($CDCl_3$) : 3,93 (d, C$\underline{H}_3$, Kopplung mit F).

Weiteres Eluieren mit n-Hexan/Aethylacetat (9 : 1) ergibt reines 3-(o-Fluorphenyl)-1-methyl-5-(o-trifluormethyl-phenyl)-1H-1,2,4-triazol, Smp. 104-107°C, $^1$H-NMR ($CDCl_3$) : 3,82 (s, C$\underline{H}_3$).

In analoger Weise erhält man ausgehend von

– 3-(o-Chlorphenyl)-5-(o-trifluormethyl-phenyl)-1H-1,2,4-triazol und Methyljodid ein Gemisch der isomeren N-Methyltriazole und daraus das reine 5-(o-Chlorphenyl)-1-methyl-3-(o-trifluormethyl-phenyl)-1H-1,2,4-triazol als harzartiges Produkt, $^1$H-NMR($CDCl_3$): 3,86 (s, C$\underline{H}_3$) ; und das reine 3-(o-Chlorphenyl)-1-methyl-5-(o-trifluormethyl-phenyl)-1H-1,2,4-triazol, Smp. 90-92°C, $^1$H-NMR($CDCl_3$) : 3,78 (s, C$\underline{H}_3$).

– 3-(o-Chlorphenyl)-5-(o-trifluormethyl-phenyl)-1H-1,2,4-triazol und Aethylbromid ein Gemisch der isomeren N-Aethyltriazole und daraus das reine 1-Aethyl-5-(o-chlorphenyl)-3-(o-trifluormethyl-phenyl)-1H-1,2,4-triazol als harzartiges Produkt, $^1$H-NMR($CDCl_3$) : 1,47 (t, $CH_2C\underline{H}_3$), 4,10 (q, $C\underline{H}_2CH_3$) ; und das reine 1-Aethyl-3-(o-chlorphenyl)-5-(o-trifluormethyl-phenyl)-1H-1,2,4-triazol ebenfalls als harzartiges Produkt, $^1$H-NMR($CDCl_3$) : 1,45 (t, $CH_2C\underline{H}_3$), 4,04 (q, $C\underline{H}_2CH_3$).

– 3-(o-Chlorphenyl)-5-(o-trifluormethyl-phenyl)-1H-1,2,4-triazol und Allylbromid ein 1 : 1-Gemisch von 1-Allyl-5-(o-chlorphenyl)-3-(o-trifluormethyl-phenyl)-1H-1,2,4-triazol und 1-Allyl-3-(o-chlorphenyl)-5-(o-trifluormethyl-phenyl)-1H-1,2,4-triazol als Oel, $^1$H-NMR($CDCl_3$) : 4,63 und 4,71 (m, $C\underline{H}_2CH{=}CH_2$), 5,09 und 5,22 (m, $CH_2CH{=}C\underline{H}_2$), 5,91-6,01 (m, $CH_2C\underline{H}{=}CH_2$) ; sowie die entsprechenden 1-Propenyltriazole, nämlich ein 1 : 1-Gemisch von 5-(o-Chlorphenyl)-1-(1-propenyl)-3-(o-trifluormethyl-phenyl)-1H-1,2,4-triazol und 3-(o-Chlorphenyl)-1-(1-propenyl)-5-(o-trifluormethyl-phenyl)-1H-1,2,4-triazol, wobei diese zusätzlich als E/Z-Gemisch vorliegen ; $^1$H-NMR($CDCl_3$) : 1,77, 1,81, 2,0 und 2,10 (jeweils dd, $C\underline{H}_3CH{=}CH$).

– 3-(o-Chlorphenyl)-5-(o-trifluormethyl-phenyl)-1H-1,2,4-triazol und n-Propyljodid ein 11 : 9-Gemisch von 5-(o-Chlorphenyl)-1-(n-propyl)-3-(o-trifluormethyl-phenyl)-1H,1,2,4-triazol und 3-(o-Chlorphenyl)-1-(n-propyl)-5-(o-trifluormethyl-phenyl)-1H-1,2,4-triazol als Oel, $^1$H-NMR($CDCl_3$) : 0,84 und 0,89 (t, $C\underline{H}_3CH_2$), 1,85-1,96 (m, $CH_3C\underline{H}_2CH_2$), 4,02 und 3,94 (t, $CH_3CH_2C\underline{H}_2N$).

– 3-(o-Chlorphenyl)-5-(o-trifluormethyl-phenyl)-1H-1,2,4-triazol und Isopropyljodid ein 11 : 9-Gemisch von 5-(o-Chlorphenyl)-1-isopropyl-3-(o-trifluormethyl-phenyl)-1H-1,2,4-triazol und 3-(o-Chlorphenyl)-1-isopropyl-5-(o-trifluormethyl-phenyl)-1H-1,2,4-triazol als Feststoff, Smp. 82-85°C.

– 3,5-Di(o-trifluormethyl-phenyl)-1H-1,2,4-triazol und Methyljodid das 1-Methyl-3,5-di(o-trifluormethyl-phenyl)-1H-1,2,4-triazol, Smp. 108-111°C.

– 3-(o-Tolyl)-5-(o-trifluormethyl-phenyl)-1H-1,2,4-triazol und Methyljodid ein 1 : 1-Gemisch von 1-Methyl-3-(o-tolyl)-5-(o-trifluormethyl-phenyl)-1H-1,2,4-triazol und 1-Methyl-5-(o-tolyl)-3-(o-trifluormethyl-phenyl)-1H-1,2,4-triazol als Oel, $^1$H-NMR($CDCl_3$) : 2,3 und 2,63 (s, C-C$\underline{H}_3$),3,76 und 3,85 (s, N-C$\underline{H}_3$), 7,2-8,2 (8 aromatische H).

– 3-(o-Fluorphenyl)-5-(o-trifluormethyl-phenyl)-1H-1,2,4-triazol und Aethylbromid ein Gemisch der isomeren N-Aethyltriazole und daraus das reine 1-Aethyl-3-(o-fluorphenyl)-5-(o-trifluormethyl-phenyl)-1H-1,2,4-triazol Smp. 72-74°C ; und das reine 1-Aethyl-5-(o-fluorphenyl)-3-(o-trifluormethyl-phenyl)-1H-1,2,4-triazol, $^1$H-NMR($CDCl_3$) : 1,51 (t, C$\underline{H}_3$), 4,23 (q, C$\underline{H}_2$), 7,05-8,1 (8 aromatische H).

– 3-(o,o′-Dichlorphenyl)-5-(o-trifluormethyl-phenyl)-1H-1,2,4-triazol und Methyljodid ein Gemisch der isomeren N-Methyltriazole und daraus das reine 5-(o,o′-Dichlorphenyl)-1-methyl-3-(o-trifluormethyl-phenyl)-1H-1,2,4-triazol, Smp. 101-103°C ; und das reine 3-(o,o′-Dichlorphenyl)-1-methyl-5-(o-trifluormethyl-phenyl)-1H-1,2,4-triazol als harzartiges Produkt, Massenspektrum : 371 (55), 352 (2), 200 (100), 181 (12), 152 (55).

Beispiel 2

Eine Lösung von 2 g o,o'-Difluorbenzimidsäure-äthylester in 15 ml Diäthyläther wird mit 1,09 g Triäthylamin versetzt und mittels eines Eisbads auf 0-5°C gekühlt. Dann gibt man 2,25 g o-Trifluormethyl-benzoylchlorid in 5 ml Diäthyläther während 10 Minuten tropfenweise zu und rührt das Reaktionsgemisch eine Stunde noch bei 0-5°C weiter. Zur Aufarbeitung giesst man das Gemisch auf Eiswasser und extrahiert das wässrige Gemisch dreimal mit Diäthyläther. Die vereinigten Extrakte werden der Reihe nach mit Wasser, halbgesättigter Natriumchloridlösung und gesättigter Natriumchloridlösung gewaschen, über wasserfreiem Natriumsulfat getrocknet und unter vermindertem Druck eingedampft. Auf diese Weise erhält man den rohen N-(o-Trifluormethyl-benzoyl) -o,o'-difluorbenzimidsäure-äthylester (4,94 g) als feuchten gelben Feststoff, Smp. 64-69°C.

Ohne weitere Reinigung werden 4,0 g des obigen Rohpro-dukts in Methylenchlorid vorgelegt und auf 10°C gekühlt. Man lässt 0,54 g Methylhydrazin innert 3 Minuten unter Rühren zutropfen, rührt 30 Minuten bei Raumtemperatur weiter und erwärmt dann während 30 Minuten auf Rückflusstemperatur. Es werden anschliessend weitere 0,54 g Methylhydrazin zugesetzt und wiederum während 30 Minuten auf Rückflusstemperatur erwärmt, um verbleibendes Ausgangsmaterial reagieren zu lassen. Man giesst das Gemisch auf Wasser und extrahiert zweimal mit Methylenchlorid, wäscht die vereinigten Extrakte mit Wasser nach, trocknet sie über wasserfreiem Natriumsulfat und dampft sie unter vermindertem Druck ein. Der ölige Rückstand wird durch Chromatographie an Kieselgel mit n-Hexan/Aethylacetat (4 : 1) gereinigt. Auf diese Weise erhält man das 3-(o,o'-Difluorphenyl)-1-methyl-5-(o-trifluormethyl-phenyl)-1H-1,2,4-triazol als Feststoff, Smp. 103-107°C.

In analoger Weise erhält man ausgehend von :

– o-Brombenzimidsäure-äthylester und o-Trifluormethyl-benzoylchlorid den rohen N-(o-Trifluormethyl-benzoyl)-o-brombenzimidsäure-äthylester und daraus mit Methylhydrazin das 3-(o-Bromphenyl)-1-methyl-5-(o-trifluormethyl-phenyl)-1H -1,2,4-triazol, Smp. 90-94°C.

– o,p-Dichlorbenzimidsäure-äthylester und o-Trifluormethyl-benzoylchlorid den N-(o-Trifluormethyl-benzoyl)-2,4-dichlorbenzimidsäure-äthylester und daraus mit Methylhydrazin das 3-(o,p-Dichlorphenyl)-1-methyl-5-(o-trifluormethyl-phenyl)-1H-1,2,4-triazol, Smp. 101-103°C ; $^1$H-NMR($CDCl_3$) : 3,80 (s, $C\underline{H}_3$), 7,2-8,05 (7 aromatische H).

– o-Methoxybenzimidsäure-äthylester und o-Trifluormethyl-benzoylchlorid den N-(o-Trifluormethyl-benzoyl)-o-methoxybenzimidsäure-äthylester und daraus mit Methylhydrazin das 3-(o-Anisyl)-1-methyl-5-(o-trifluormethyl-phenyl)-1H- 1,2,4-triazol, Smp. 74-78°C.

– 2,5-Dichlorbenzimidsäure-äthylester und o-Trifluormethyl-benzoylchlorid den N-(o-Trifluormethyl-benzoyl)-2,5-dichlorbenzimidsäure und daraus mit Methylhydrazin das 3-(2,5-Dichlorphenyl)-1-methyl-5-(o-trifluormethyl-phenyl)-1H-1,2,4-triazol, Smp. 60-63°C.

– Nikotinimidsäure-methylester und o-Trifluormethyl-benzoylchlorid den N-(o-Trifluormethyl-benzoyl)-3-pyridylcarboximidsäure-methylester und daraus mit Methylhydrazin das 1-Methyl-3-(3-pyridyl)-5-(o-trifluormethyl-phenyl)-1H-1,2,4-triazol, $^1$H-NMR($CDCl_3$) : 3,76 (s, $C\underline{H}_3$), 7,3-8,0 (C$\underline{H}$-4 und 4 aromatische H), 8,43 (ddd, C$\underline{H}$-5), 8,68 (dd, C$\underline{H}$-6), 9,41 (d, C$\underline{H}$-2).

– 2-Chlor-6-fluorbenzimidsäure-äthylester und o-Trifluormethyl-benzoylchlorid den N-(o-Trifluormethyl-benzoyl)-2-chlor-6-fluorbenzimidsäure-äthylester und daraus mit Methylhydrazin das 3-(2-Chlor-6-fluorphenyl)-1-methyl-5-(o-trifluormethyl-phenyl)-1H-1,2,4-triazol, Smp. 80-84°C.

– o-Chlorbenzimidsäure-äthylester und o-Trifluormethyl-benzoylchlorid den N-(o-Trifluormethyl-benzoyl)-o-chlorbenzimidsäure-äthylester, Smp. 64-65°C, und daraus mit 2,2,2-Trifluoräthylhydrazin das 3-(o-Chlorphenyl)-1-(2,2,2-trifluoräthyl)-5-(o-trifluormethyl-phenyl)-1H-1,2,4-triazol, $^1$H-NMR($CDCl_3$) : 4,62 (q, $C\underline{H}_2CF_3$), 7,3-8,1 (8 aromatische H).

– 2-Pyridylcarboximidsäure-äthylester und o-Trifluormethyl-benzoylchlorid den N-(o-Trifluormethyl-benzoyl)-2-pyridylcarboximidsäure-äthylester und daraus mit Methylhydrazin das 1-Methyl-3-(2-pyridyl)-5-(o-trifluormethyl-phenyl)-1H-1,2,4-triazol, $^1$H-NMR($CDCl_3$) : 3,8 (s, $C\underline{H}_3$), 7,15-8,0 (6H), 8,2 (m, 1H), 8,73 (m, 1H).

– Isonikotinimidsäure-methylester und o-Trifluormethyl-benzoylchlorid den rohen N-(o-Trifluormethyl-benzoyl)-4-pyridylcarboximidsäure-methylester und daraus mit Methylhydrazin das 1-Methyl-3-(4-pyridyl)-5-(o-trifluormethyl-phenyl)-1H -phenyl)-1H -1,2,4-triazol, Smp. 43-146°C.

– 2-Chlor-4-fluorbenzimidsäure-äthylester und o-Trifluormethyl-benzoylchlorid den N-(o-Trifluormethyl-benzoyl)-2-chlor-4-fluorbenzimidsäure-äthylester und daraus mit Methylhydrazin das 3-(2-Chlor-4-fluorphenyl)-1-methyl-5-(o-trifluormethyl-phenyl)-1H -1,2,4-triazol, Smp. 67-69°C.

– o-Jodbenzimidsäure-äthylester und o-Trifluormethylbenzoylchlorid den rohen N-(o-Trifluormethyl-benzoyl)-o-jodbenzimidsäure-äthylester und daraus mit Methylhydrazin das 3-(o-Jodphenyl)-1-methyl-5-(o-trifluormethyl-phenyl)-1H-1,2,4-triazol, Smp. 84-88°C.

– o,p-Difluorbenzimidsäure-äthylester und o-Trifluormethyl-benzoylchlorid den N-(o-Trifluormethyl-benzoyl)-o,p-difluorbenzimidsäure-äthylester und daraus mit Methylhydrazin das 3-(o,p-Difluorphenyl)-1-methyl-5-(o-trifluormethylphenyl)-1H -1,2,4-triazol, Smp. 54-56°C.

– 4-Chlor-2-fluorbenzimidsäure-äthylester und o-Trifluormethyl-benzoylchlorid den N-(o-Trifluormethyl-benzoyl)-2-fluor-4-chlorbenzimidsäure-äthylester und daraus mit Methylhydrazin das 3-(4-Chlor-2-fluorphenyl)-1-methyl-5-(o-trifluormethyl-phenyl)-1H -1,2,4-triazol, Smp. 109,5-112°C.

– 3-Chlor-2-fluorbenzimidsäure-äthylester und o-Trifluormethyl-benzoylchlorid den N-(o-Trifluormethyl-benzoyl)-2-fluor-3-chlorbenzimidsäure-äthylester und daraus mit Methylhydrazin das 3-(3-Chlor-2-fluorphenyl)-1-methyl-5-(o-trifluormethyl-phenyl)-1H -1,2,4-triazol, Smp. 37-140°C.

– o,o'-Difluorbenzimidsäure-äthylester und o-Trifluormethyl-benzoylchlorid den N-(o-Trifluormethyl-benzoyl)-o,o'-difluorbenzimidsäure-äthylester und daraus mit Aethylhydrazin das 1-Aethyl-3-(o,o'-difluorphenyl)-5-(o-trifluormethylphenyl)-1H -1,2,4-triazol, Smp. 86-89°C.

– 2,3-Dichlorbenzimidsäure-äthylester und o-Trifluormethyl-benzoylchlorid den N-(o-Trifluormethyl-benzoyl)-2,3-dichlorbenzimidsäure-äthylester und daraus mit Methylhydrazin das 3-(2,3-Dichlorphenyl)-1-methyl-5-(o-trifluormethylphenyl)-1H -1,2,4-triazol, Smp. 86-88°C.

– o-Chlorbenzimidsäure-äthylester und 4-Trifluormethylnikotinsäurechlorid den N-(4-Trifluormethyl-nikotinoyl)-2-chlorbenzimidsäure-äthylester und daraus mit Methylhydrazin das 3-(o-Chlorphenyl)-1-methyl-5-(4-trifluormethyl-3-pyridyl)-1H -1,2,4-triazol, $^1H$-NMR($CDCl_3$) : 3,85 (s, N-$CH_3$), 7,6-8,1 (5 aromatische H), 8,9-9,2 (2 aromatische H) ; Massenspektrum : 338 (100), 319 (5), 166 (94), 138 (95).

– o-Cyanobenzimidsäure-äthylester und o-Trifluormethylbenzoylchlorid den N-(o-Trifluormethyl-benzoyl)-o-cyanobenzimidsäure-äthylester und daraus mit Methylhydrazin das 3-(o-Cyanophenyl)-1-methyl-5-(o-trifluormethyl-phenyl)-1H -1,2,4-triazol, Smp. 96,5-99°C.

## Beispiel 3

Ein Gemisch von 1,1 g 3-(o-Chlorphenyl)-1-(2-hydroxyäthyl)-5 -(o-trifluormethyl-phenyl)-1H-1,2,4-triazol, 1,09 g Thionylchlorid und 5 ml Toluol wird während 4 Stunden bei Rückflusstemperatur erhitzt. Dann dampft man das Reaktionsgemisch ein, nimmt den Rückstand in Diäthyläther auf, wäscht die Lösung zweimal mit 5%iger Natriumbicarbonatlösung, trocknet sie über wasserfreiem Magnesiumsulfat und dampft sie ein. Man erhält auf diese Weise das 1-(2-Chloräthyl)-3-(o-chlorphenyl)-5-(o-trifluormethyl-phenyl)-1H-1,2,4-triazol, Smp. 66-68°C.

## II. Herstellung der Ausgangsmaterialien der Formel II :

## Beispiel 4

Eine Lösung von 2,01 g Hydrazinhydrat in 6 ml Aethanol wird auf Rückflusstemperatur erhitzt. Man lässt 3,63 g Azin von o-Fluorbenzoylchlorid und o-Trifluormethyl-benzoylchlorid gelöst in 30 ml warmem Aethanol innert 15 Minuten zutropfen und lässt 45 Minuten bei Rückflusstemperatur reagieren. Dann destilliert man den Alkohol ab, versetzt den öligen Rückstand innert 10 Minuten mit 25 ml konzentrierter Salzsäure (mindestens 32%) und rührt das Gemisch eine Stunde bei 80°C. Alsdann wird das Gemisch mittels eines Eisbades gekühlt und durch Zugabe von 30 ml Ammoniaklösung (ca. 25%) basisch gestellt. Dreimaliges Extrahieren mit Methylenchlorid, zweimaliges Waschen mit Wasser, Trocknen über wasserfreiem Natriumsulfat und Abdampfen des Lösungsmittels ergibt 3,21 g rohes 4-Amino-3-(o-fluorphenyl)-5-(o-trifluormethyl-phenyl)-4H-1,2,4-triazol.

Ohne weitere Reinigung wird das obige Rohprodukt in 42 ml Eisessig gelöst, und der Lösung wird eine Lösung von 1,87 g Natriumnitrit in 8 ml Wasser bei 15°C innert 15 Minuten zugetropft. Anschliessend wird eine Stunde nachgerührt und durch Zugabe von 42 ml Ammoniaklösung schwach basisch gestellt. Das ausgefallene Produkt wird abgenutscht und bei 70°C/200 mmHg getrocknet. Man erhält auf diese Weise reines 3-(o-Fluorphenyl)-5-(o-trifluormethylphenyl)-1H-1,2,4-triazol, Smp. 153-155°C.

In analoger Weise erhält man ausgehend von

– dem Azin von o-Chlorbenzoylchlorid und o-Trifluormethyl-benzoylchlorid und Hydrazinhydrat das 4-Amino-3-(o-chlorphenyl)-5-(o-trifluormethyl-phenyl)-4H-1,2,4-triazol und daraus durch Desaminierung das 3-(o-Chlorphenyl)-5-(o-trifluormethyl-phenyl)-1H-1,2,4-triazol, Smp. 172-175°C.

– o-Trifluormethyl-benzoylazindichlorid und Hydrazinhydrat das 4-Amino-3,5-di(o-trifluormethyl-phenyl)-4H-1,2,4-triazol und daraus durch Desaminierung das 3,5-Di(o-trifluormethyl-phenyl)-1H-1,2,4-triazol, Smp. 189,5-192°C.

– dem Azin von o-Toluylchlorid und o-Trifluormethyl-benzoylchlorid und Hydrazinhydrat das 4-Amino-3-(o-tolyl)-5-(o-trifluormethyl-phenyl)-4H-1,2,4-triazol und daraus durch Desaminierung das 3-(o-Tolyl)-5-(o-trifluormethyl-phenyl)-1H-1,2,4-triazol.

– dem Azin von o,o'-Dichlorbenzoylchlorid und o-Trifluormethyl-benzoylchlorid und Hydrazinhydrat das 4-Amino-3-(o,o'-dichlorphenyl)-5-(o-trifluormethyl-phenyl)-4H-1,2,4-triazol und daraus durch Desaminierung das 3-(o,o'-Dichlorphenyl)-5-(o-trifluormethyl-phenyl)-1H-1,2,4-triazol.

Beispiel 5

Zu einer Lösung von 2,64 g Natriumhydroxid in 34 ml Wasser werden 9,24 g o-Fluorbenzoylhydrazid unter Rühren zugesetzt. Man kühlt dann das Gemisch auf 0-5°C und lässt 13,76 g o-Trifluormethyl-benzoylchlorid während 20 Minuten zutropfen. Das Reaktionsgemisch wird noch eine Stunde bei 5-10°C gerührt und anschliessend mit 30 ml Wasser verdünnt. Man nutscht den resultierenden Kristallbrei ab, wäscht ihn mit etwas Wasser auf der Nutsche nach und trocknet ihn bei 80°C/180 mm Hg. Auf diese Weise erhält man das 1-(o-Fluorbenzoyl)-2-(o-trifluormethyl-benzoyl)-hydrazin, Smp. 171-173°C.

17,4 g des obigen Produktes werden portionenweise innert 20 Minuten zu einer auf 110°C erwärmten Lösung von 33,3 g Phosphorpentachlorid in 50 ml 1,2-Dichlorbenzol gegeben. Man lässt eine Stunde bei 110°C reagieren und destilliert dann das Lösungsmittel und das überschüssige Phosphorpentachlorid am Wasserhahnvakuum ab. Der erkaltete Rückstand wird mit Wasser versetzt und dreimal mit Aethylacetat extrahiert. Die vereinigten Extrakte werden zweimal mit Wasser gewaschen, über wasserfreiem Natriumsulfat getrocknet und unter vermindertem Druck eingedampft, und der feste Rückstand wird durch Chromatographie an Kieselgel mit n-Hexan/Aethylacetat (19 : 1) gereinigt. Auf diese Weiser erhält man das reine Azin von o-Fluorbenzoylchlorid und o-Trifluormethyl-benzoylchlorid, Smp. 73-75°C.

[Unter nachträglicher Verwendung von n-Hexan/Aethylacetat (2 : 1) als Laufmittel erhält man das in kleinerer Menge als Nebenprodukt entstehende 2-(o-Fluorphenyl)-5-(o-trifluormethyl-phenyl)-1,3,4-oxadiazol, Smp. 68-69°C (nach Umkristallisation aus n-Hexan/Aethylacetat).]

In analoger Weise erhält man ausgehend von

– o-Chlorbenzoylhydrazid und o-Trifluormethyl-benzoylchlorid das 1-(o-Chlorbenzoyl)-2-(o-trifluormethyl-benzoyl)-hydrazin, Smp. 241-243°C, und daraus durch Chlorierung das Azin von o-Chlorbenzoylchlorid und o-Trifluormethyl-benzoylchlorid, Smp. 81,5-82,5°C (nach Umkristallisation aus Isopropanol).

– o-Trifluormethyl-benzoylhydrazid und o-Trifluormethyl-benzoylchlorid das rohe 1,2-Di(o-trifluormethyl-benzoyl)-hydrazin und daraus durch Chlorierung das o-Trifluormethyl-benzoylazin-dichlorid, Smp. 100-104°C.

– o-Toluylhydrazid und o-Trifluormethyl-benzoylchlorid das rohe 1-(o-Toluyl)-2-(o-trifluormethyl-benzoyl)-hydrazin und daraus durch Chlorierung das Azin von o-Toluylchlorid und o-Trifluormethyl-benzoylchlorid, Smp. 55-57°C.

– o,o'-Dichlorbenzoylhydrazid und o-Trifluormethyl-benzoylchlorid das 1-(o,o'-Dichlorbenzoyl)-2-(o-trifluormethyl-benzoyl)-hydrazin und daraus durch Chlorierung das Azin von o,o'-Dichlorbenzoylchlorid und o-Trifluormethyl-benzoylchlorid.

III. Herstellung der Ausgangsmaterialien der Formel VI :

Beispiel 6

Eine Lösung von 39,2 g Triäthyloxonium-tetrafluorborat in 325 ml Methylenchlorid wird mit 32,5 g o-Trifluormethyl-benzamid versetzt. Nach 48-stündigem Rühren bei Raumtemperatur wird das Reaktionsgemisch auf ein Gemisch von 10%-iger Natriumcarbonatlösung und Eis gegossen und das ganze dreimal mit Methylenchlorid extrahiert. Die vereinigten Extrakte werden dann zweimal mit 10%-iger Natriumcarbonatlösung nachgewaschen, über wasserfreiem Natriumsulfat getrocknet und unter vermindertem Druck eingedampft. Der ölige, braune Rückstand wird am Hochvakuum destilliert. Man erhält den reinen o-Trifluormethyl-benzimidsäure-äthylester, Sdp. 67°C/0,15 mmHg.

In analoger Weise erhält man ausgehend von

– 2,5-Dichlorbenzamid und Triäthyloxonium-tetrafluorborat den 2,5-Dichlorbenzimidsäure-äthylester, Smp. 40-43°C.

– 4-Chlor-2-fluorbenzamid und Triäthyloxonium-tetrafluoroborat den 4-Chlor-2-fluorbenzimidsäure-äthylester als Oel ; Massenspektrum : 202 (3,5), 201 (1), 200 (10), 182 (2), 173 (27), 157 (100), 156 (75).

– o-Jodbenzamid und Triäthyloxonium-tetrafluoroborat den o-Jodbenzimidsäure-äthylester als Oel ; $^1$H-NMR($CDCl_3$) : 1,42 (t, $C\underline{H}_3$), 4,38 (q, $C\underline{H}_2$), 6,8-7,6 (3 aromatische H + $N\underline{H}$), 7,8-8,05 (1 aromatischer

H).

– 3-Chlor-2-fluorbenzamid und Triäthyloxonium-tetrafluoroborat den 3-Chlor-2-fluorbenzimidsäure-äthylester als Oel ; Massenspektrum : 200 (10), 173 (25), 166 (15), 157 (100), 156 (73).

– o,p-Difluorbenzamid und Triäthyloxonium-tetrafluoroborat den o,p-Difluorbenzimidsäure-äthylester als Oel ; Massenspektrum : 184 (10), 166 (3), 157 (36), 141 (100), 140 (95).

– 2,3-Dichlorbenzamid und Triäthyloxonium-tetrafluoroborat den 2,3-Dichlorbenzimidsäure-äthylester als Oel ; Massenspektrum : 216 (16), 189 (20), 182 (9), 173 (100), 172 (56).

– 2-Chlor-4-fluorbenzamid und Triäthyloxonium-tetrafluoroborat den 2-Chlor-4-fluorbenzimidsäure-äthylester als Oel ; $^1$H-NMR($CDCl_3$) : 1,43 (t, $C\underline{H}_3$), 4,40 (q, $C\underline{H}_2$), 6,9-7,8 (3 aromatische H + N$\underline{H}$).

## IV. Herstellung der Ausgangsmaterialien der Formel IX :

### Beispiel 7

Analog dem in Beispiel 2 beschriebenen Verfahren erhält man ausgehend aus o-Chlorbenzimidsäure-äthylester und o-Trifluormethyl-benzoylchlorid den N-(o-Trifluormethyl-benzoyl)-o-chlorbenzimidsäure-äthylester, Smp. 64-65°C, und daraus mittels 2-Hydroxyäthylhydrazid das 3-(o-Chlorphenyl)-1-(2-hydroxyäthyl)-5-(o-trifluormethyl-phenyl)-1H-1,2,4-triazol, Smp. 115°C.

## V. Formulierungsbeispiele :

### Beispiel 8

Ein emulgierbares Konzentrat hat folgende Zusammensetzung :

| | | g/Liter |
|---|---|---|
| Verbindung der Formel I (Wirkstoff) | | 250 |
| Nonylphenol-(10)äthyoxylat | (Emulgatoren) | 50 |
| Calcium-Dodecylbenzolsulfonat | | 25 |
| Gemisch aus Mono-, Di- und Tri(nieder alkyl)benzolen (Lösungsmittel) | ad | 1000 ml |

Der Wirkstoff und die beiden Emulgatoren werden im Lösungsmittel bei Raumtemperatur gelöst. Nach Verdünnung mit Wasser ergibt das so entstandene emulgierbare Konzentrat eine Emulsion, die sich als Spritzbrühe gut eignet.

### Beispiel 9

Ein Spritzpulver hat folgende Zusammensetzung :

| | | Gewichtsprozent |
|---|---|---|
| Verbindung der Formel I (Wirkstoff) | | 50 |
| Natrium-laurylsulfat (Netz-/Dispergiermittel) | | 1 |
| Natrium-lignosulfonat (Dispergiermittel) | | 2 |
| Hydratisierte Kieselsäure (ca. 87% $SiO_2$) | (inerte, pulver- | 5 |
| Kaolin (hauptsächlich $Al_2(Si_2O_5)(OH)_4$) | förmige Träger-stoffe) | 42 |
| | | 100 |

Der Wirkstoff wird mit den übrigen Formulierungskomponenten in einer geeigneten Vorrichtung homogen vermischt. Das entstandene Pulver wird nun in einem geeigneten Mahlaggregat (z.B. Stiften-, Hammer-, Kugel- oder Luftstrahlmühle) auf eine für eine optimale biologische Wirksamkeit notwendige Teilchengrösse feingemahlen und hernach nochmals gemischt. Das nun vorliegende Spritzpulver wird durch Wasser spontan benetzt und ergibt gut schwebefähige, gebrauchsfertige Spritzbrühen.

## Ansprüche

### Patentansprüche für die Vertragsstaaten : BE, CH, DE, FR, GB, IT, LI, NL, SE

1. Verbindungen der allgemeinen Formel

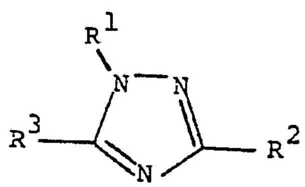

I

worin
$R^1$ $C_{1-4}$-Alkyl, $C_{1-4}$-Halogenalkyl oder $C_{2-4}$-Alkenyl,
$R^2$ mit 1 bis 3 Chlor-, Brom- und/oder Jodatomen, 1 bis 5 Fluoratomen, 1 oder 2 $C_{1-4}$-Alkylgruppen, 1 oder 2 Halogenmethylgruppen, 1 oder 2 $C_{1-2}$-Alkoxygruppen, oder 2 $C_{1-2}$-Halogenalkoxygruppen, einer Methylthiogruppe, einer Cyanogruppe und/oder einer Nitrogruppe substituiertes Phenyl oder gegebenenfalls mit 1 bis 3 Chloratomen und/oder einer Methylgruppe substituiertes 2-, 3- oder 4-Pyridyl
und $R^3$ o-Trifluormethyl-phenyl oder 4-Trifluormethyl-3-pyridyl bedeuten, sowie die Säureadditionssalze der Verbindungen der Formel I.

2. Verbindungen nach Anspruch, 1 worin $R^2$ mit 1 bis 3 Fluor-, Chlor-, Brom- und/oder Jodatomen, 1 oder 2 $C_{1-4}$-Alkylgruppen, 1 oder 2 Trifluormethylgruppen, 1 oder 2 $C_{1-2}$-Alkoxygruppen, einer Methylthiogruppe, einer Cyanogruppe und/oder einer Nitrogruppe substituiertes Phenyl oder gegebenenfalls mit 1 bis 3 Chloratomen und/oder einer Methylgruppe substituiertes 2-, 3- oder 4-Pyridyl und $R^3$ o-Trifluormethyl-phenyl bedeuten.

3. Verbindungen nach Anspruch 1 oder 2, worin $R^1$ $C_{1-4}$-Alkyl bedeutet.

4. Verbindungen nach eine der Ansprüche 1 bis 3, worin $R^2$ substituiertes phenyl bedeutet, das zumindest einen Substituenten in einer ortho-Stellung aufweist.

5. Verbindungen nach Anspruch 4, worin $R^2$ o-Halogenphenyl oder o,o'-Dihalogenphenyl bedeutet.

6. Verbindungen nach Anspruch 4, worin $R^2$ o,p-Dihalogenphenyl bedeutet.

7. Eine Verbindung nach Anspruch 2, ausgewählt aus :
3-(o-Chlorphenyl)-1-methyl-5-(o-trifluormethyl-phenyl)-1H-1,2,4-triazol,
3-(o,o'-Difluorphenyl)-1-methyl-5-(o-trifluormethyl-phenyl)-1H-1,2,4-triazol,
3-(o-Bromphenyl)-1-methyl-5-(o-trifluormethyl-phenyl)1H-1,2,4-triazol,
3-(2-Chlor-6-fluorphenyl)-1-ethyl-5-(o-trifluormethyl-phenyl)-1H-1,2,4-triazol,
3-(o,p-Dichlorphenyl)-1-methyl-5-(o-trifluormethyl-phenyl)-1H-1,2,4-triazol und
3-(2-Chlor-4-fluorphenyl)-1-methyl-5-(o-trifluormethyl-phenyl)-1H-1,2,4-triazol.

8. Eine Verbindung nach Anspruch 2, ausgewählt aus :
3-(o-Fluorphenyl)-1-methyl-5-(o-trifluormethyl-phenyl)-1H-1,2,4-triazol,
1-Aethyl-3-(o-chlorphenyl)-5-(o-trifluormethyl-phenyl)-1H–1,2,4-triazol,
1-Allyl-3-(o-chlorphenyl)-5-(o-trifluormethyl-phenyl)-1H-1,2,4-triazol,
3-(o-Chlorphenyl)-1-(1-propenyl)-5-(o-trifluormethyl-phenyl)-1H-1, 2,4-triazol,
3-(o-Chlorphenyl)-1-(n-propyl)-5-(o-trifluormethyl-phenyl)-1H-1, 2,4-triazol,
3-(o-Chlorphenyl)-1-isopropyl-5-(o-trifluormethyl-phenyl)-1H-1,2,4-triazol,
1-Methyl-3,5-di(o-trifluormethyl-phenyl)-1H-1,2,4-triazol,
1-Methyl-3-(o-tolyl)-5-(o-trifluormethyl-phenyl)-1H-1,2,4-triazol,
1-Aethyl-3-(o-fluorphenyl)-5-(o-trifluormethyl-phenyl)-1H-1,2,4-triazol,
3-(o,o'-Dichlorphenyl)-1-methyl-5-(o-trifluormethylphenyl)-1H-1, 2,4-triazol,

3-(o-Anisyl)-1-methyl-5-(o-trifluormethyl-phenyl)-1H-1,2,4-triazol,
3-(2,5-Dichlorphenyl)-1-methyl-5-(o-trifluormethyl-phenyl)-1H-1,2,4-triazol,
1-Methyl-3-(3-pyridyl)-5-(o-trifluormethyl-phenyl)-1H-1,2,4-triazol,
3-(o-Chlorphenyl)-1-(2,2,2-trifluoräthyl)-5-(o-trifluormethyl-phenyl)-1H-1,2,4-triazol,
1-Methyl-3-(2-pyridyl)-5-(o-trifluormethyl-phenyl)-1H-1,2,4-triazol,
1-Methyl-3-(4-pyridyl)-5-(o-trifluormethyl-phenyl)-1H-1,2,4-triazol,
3-(o-Jodphenyl)-1-methyl-5-(o-trifluormethyl-phenyl)-1H-1,2,4-triazol,
3-(o,p-Difluorphenyl)-1-methyl-5-(o-trifluorethyl-phenyl)-1H-1,2,4-triazol,
3-(4-Chlor-2-fluorphenyl)-1-methyl-5-(o-trifluormethyl-phenyl)-1H-1,2,4-triazol,
3-(3-Chlor-2-fluorphenyl)-1-methyl-5-(o-trifluormethyl-phenyl)-1H-1,2,4-triazol,
3-(2,3-Dichlorphenyl)-1-methyl-5-(o-trifluormethyl-phenyl)-1H-1,2,4-triazol,
3-(o-Cyanophenyl)-1-methyl-5-(o-trifluormethyl-phenyl)-1H-1,2,4-triazol und
1-(2-Chloräthyl)-3-(o-chlorphenyl)-5-(o-trifluormethyl-phenyl)-1H-1,2,4-triazol.

9. Eine Verbindung nach Anspruch, ausgewählt aus :
1-Aethyl-3-(o,o'-difluorphenyl)-5-(o-trifluormethyl-phenyl)-1H-1,2,4-triazol und
3-(o-Chlorphenyl)-1-methyl-5-(4-trifluormethyl-3-pyridyl)-1H-1,2,4-triazol.

10. Verbindungen nach Anspruch 1 als Wirkstoffe von Schädlingsbekämpfungsmitteln.

11. Schädlingsbekämpfungsmittel, dadurch gekennzeichnet, dass es eine wirksame Menge mindestens einer Verbindung der allgemeinen Formel I gemäss Anspruch 1 oder eines Säureadditionssalzes davon, sowie Formulierungshilfsstoffe enthält.

12. Schädlingsbekämpfungsmittel nach Anspruch 11 dadurch gekenneichenet, dass es eine wirksame Menge mindestens einer Verbindung gemäss Anspruch 2 sowie Formulierungshilfsstoffe enthält.

13. Schädlingsbekämpfungsmittel nach Anspruch 11, dadurch gekennzeichnet. dass es eine wirksame Menge mindestens einer Verbindung gemäss Anspruch 7 sowie Formulierungshilfsstoffe enthält.

14. Verfahren zur Herstellung von Verbindungen der allgemeinen Formel I gemäss Anspruch 1 und von ihren Säureadditionssalzen, dadurch gekennzeichnet, dass man

a) ein 1,2,4-Triazol der allgemeinen Formel

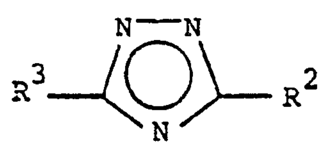 II

worin $R^2$ und $R^3$ die in Anspruch 1 angegebenen Bedeutungen besitzen, mit einer Verbindung der allgemeinen Formel

$$R^1 - X \qquad III$$

worin $R^1$ die in Anspruch 1 angegebene Bedeutung besitzt und X eine Abgangsgruppe bedeutet, umsetzt,

b) eine Verbindung der allgemeinen Formel

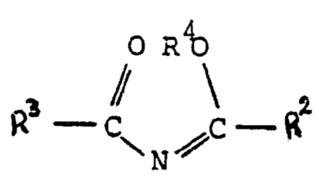 IV

worin $R^2$ und $R^3$ die in Anspruch 1 angegebenen Bedeutungen besitzen, und $R^4$ einen Niederalkylrest bedeutet, oder ein Säureadditionssalz davon, mit eine Hydrazin der allgemeinen Formel

$$R^1\text{-NH-NH}_2 \qquad V$$

worin $R^1$ die in Anspruch 1 angegebene Bedeutung besitzt, umsetzt,

c) zwecks Herstellung derjenigen Verbindungen der Formel I, in denen $R^1$ $C_{1-4}$-Alkyl oder $C_{2-4}$-Alkenyl bedeutet, einen Imidsäureester der allgemeinen Formel

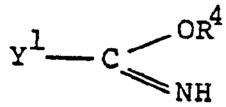

VI

worin $Y^1$ für $R^3$ oder für $R^2$ steht und $R^4$ und $R^2$ bzw. $R^3$ die in Anspruch 1 angegebenen Bedeutungen besitzen, oder ein Säureadditionssalz davon, mit einem Säurehydrazid der allgemeinen Formel

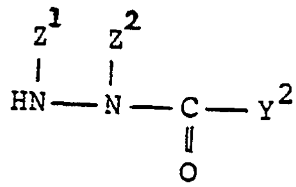

VII

worin $Y^2$ für $R^2$, $Z^1$ für $C_{1-4}$-Alkyl oder $C_{2-4}$-Alkenyl und $Z^2$ für Wasserstoff stehen (falls $Y^1$ der Formel VI $R^3$ bedeutet) oder $Y^2$ für $R^3$, $Z^1$ für Wasserstoff und $Z^2$ für $C_{1-4}$-Alkyl oder $C_{2-4}$-Alkenyl stehen (falls $Y^1$ der Formel VI $R^2$ bedeutet), wobei $R^2$ bzw. $R^3$ die in Anspruch 1 angegebene Bedeutung besitzt, zu eine Acylamidrazon umsetzt und dieses durch Erhitzen cyclisiert,

d) zwecks Herstellung derjenigen Verbindungen der Formel I, in denen $R^1$ $C_{1-4}$-Alkyl oder $C_{2-4}$-Alkenyl bedeutet, ein Diacyl-, Monothiodiacyl- bzw. Dithiodiacylamin der allgemeinen Formel

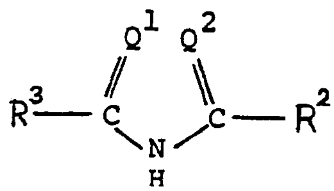

VIII

worin $R^2$ und $R^3$ die in Anspruch 1 angegebenen Bedeutungen besitzen und $Q^1$ und $Q^2$ unabhängig voneinander Sauerstoff oder Schwefel bedeuten, mit einem Hydrazin der allgemeinen Formel

$$R^{1'}\text{-NH-NH}_2 \qquad \text{V'}$$

worin $R^{1'}$ $C_{1-4}$-Alkyl oder $C_{2-4}$-Alkenyl bedeutet, umsetzt, oder

e) zwecks Herstellung derjenigen Verbindungen der Formel I, in denen $R^1$ $C_{1-4}$-Halogenalkyl bedeutet, ein 1-Hydroxyalkyl-1H-1,2,4-triazol der allgemeinen Formel

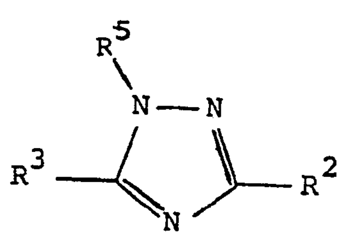

IX

worin $R^5$ $C_{1-4}$-Hydroxyalkyl bedeutet, und $R^2$ und $R^3$ die in Anspruch 1 angegebenen Bedeutungen besitzen, mit einem Halogenierungsmittel behandelt,

und erwünschtenfalls eine so erhaltene Verbindung der Formel I durch Umsetzung mit einer Säure in das entsprechende Säureadditionssalz überführt

15. Verfahren nach Anspruch 14 zur Herstellung von Verbindungen gemäss Anspruch 2.

16. Verfahren zur Bekämpfung von Schädlingen, dadurch gekennzeichnet, dass man das zu schützende Gut oder die Schädlinge selbst mit einer wirksamen Menge einer Verbindung gemäss einem der Ansprüche 1,6 und 9 bzw. eines Mittels gemäss Anspruch 11 behandelt.

17. Verfahren zur Bekämpfung von Schädlingen, dadurch gekennzeichnet, dass man das zu schützende Gut oder die Schädlinge selbst mit einer wirksamen Menge einer Verbindung gemäss einem der Ansprüche 2 bis 5, 7 und 8 bzw. eines Mittels gemäss Anspruch 12 oder 13 behandelt.

18. Verwendung einer Verbindung gemäss eine der Ansprüche 1, 6 und 9 bzw. eines Mittels gemäss Anspruch 11 zur Bekämpfung von Schädlingen.

19. Verwendung einerverbindung gemäss einem der Ansprüche 2 bis 5,7 und 8 bzw, eines Mittels gemäss Anspruch 12 oder 13 zur Bekämpfung von Schädlingen.

**Patentansprüche für Vertragsstaat : AT**

1. Schädlingsbekämpfungsmittel, dadurch gekennzeichnet, dass es eine wirksame Menge mindestens einer Verbindung der allgemeinen Formel

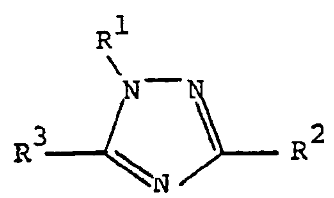

I

worin

$R^1$ $C_{1-4}$-Alkyl, $C_{1-4}$-Halogenalkyl oder $C_{2-4}$-Alkenyl,

$R^2$ mit bis 3 Chlor-, Brom- und/oder Jodatomen, 1 bis 5 Fluoratomen, 1 oder 2 $C_{1-4}$-Alkylgruppen, 1 oder 2 Halogenmethylgruppen, 1 oder 2 $C_{1-2}$-Alkoxygruppen, 1 oder 2 $C_{1-2}$-Halogenalkoxygruppen, einer Methylthiogruppe, einer Cyanogruppe und/oder einer Nitrogruppe substituiertes Phenyl oder gegebenenfalls mit 1 bis 3 Chloratomen und/oder einer Methylgruppe substituiertes 2-, 3- oder 4-Pyridyl

und $R^3$ o-Trifluormethyl-phenyl oder 4-Trifluormethyl-3-pyridyl bedeuten, oder eines Säureadditionssalzes davon, sowie Formulierungshilfsstoffe enthält.

2. Schädlingsbekämpfungsmittel nach Anspruch 1, worin $R^2$ mit bis 3 Fluor-, Chlor-, Brom- und/oder Jodatomen, 1 oder 2 $C_{1-4}$-Alkylgruppen, 1 oder 2 Trifluormethylgruppen, 1 oder 2 $C_{1-2}$-Alkoxygruppen, einer Methylthiogruppe, einer Cyanogruppe und/oder einer Nitrogruppe substituiertes Phenyl oder gegebenenfalls mit 1 bis 3 Chloratomen und/oder einer Methylgruppe substituiertes 2-, 3- oder 4-Pyridyl und $R^3$ o-Trifluormethyl-phenyl bedeuten.

3. Schädlingsbekämpfungsmittel nach Anspruch 1 oder 2, worin $R^1$ $C_{1-4}$-Alkyl bedeutet.

4. Schädlingsbekämpfungsmittel nach einem der Ansprüche 1 bis 3, worin $R^2$ substituiertes Phenyl bedeutet, das zumindest einen Substituenten in einer ortho-Stellung aufweist.

5. Schädlingsbekämpfungsmittel nach Anspruch 4, worin $R^2$ o-Halogenphenyl oder o,o'-Dihalogenphenyl bedeutet.

6. Schädlingsbekämpfungsmittel nach Anspruch 4, worin $R^2$ o,p-Dihalogenphenyl bedeutet.

7. Schädlingsbekämpfungsmittel nach Anspruch 2, dadurch gekennzeichnet, dass es eine wirksame Menge mindestens einer aus der Gruppe

3-(o-Chlorphenyl)-1-methyl-5-(o-trifluormethyl-phenyl)-1H-1,2,4-triazol,
3-(o,o'-Difluorphenyl)-1-methyl-5-(o-trifluormethyl-phenyl)-1H-1,2,4-triazol,
3-(o-Bromphenyl)-1-methyl-5-(o-trifluormethyl-phenyl)-1H-1,2,4-triazol,
3-(2-Chlor-6-fluorphenyl)-1-methyl-5-(o-trifluormethyl-phenyl)-1H-1,2,4-triazol,
3-(o,p-Dichlorphenyl)-1-methyl-5-(o-trifluormethyl-phenyl)-1H-1,2,4-triazol und
3-(2-Chlor-4-fluorphenyl)-1-methyl-5-(o-trifluormethyl-phenyl)-1H-1,2,4-triazol
ausgewählten Verbindung sowie Formulierungshilfsstoffe enthält.

8. Schädlingsbekämpfungsmittel nach Anspruch 2, dadurch gekennzeichnet, dass es eine wirksame Menge mindestens einer aus der Gruppe

3-(o-Fluorphenyl)-1-methyl-5-(o-trifluormethyl-phenyl)-1H-1,2,4-triazol,
1-Aethyl-3-(o-chlorphenyl)-5-(o-trifluormethyl-phenyl)-1H-1,2,4-triazol,
1-Allyl-3-(o-chlorphenyl)-5-(o-trifluormethyl-phenyl)-1H-1,2,4-triazol,
3-(o-Chlorphenyl)-1-(1-propenyl)-5-(o-trifluormethyl-phenyl)-1H-1,2,4-triazol,
3-(o-Chlorphenyl)-1-(n-propyl)-5-(o-trifluormethyl-phenyl)-1H-1,2,4-triazol,

3-(o-Chlorphenyl)-1-isopropyl-5-(o-trifluormethyl-phenyl)-1H-1,2,4-triazol,
1-Methyl-3,5-di(o-trifluormethyl-phenyl)-1H-1,2,4-triazol,
1-Methyl-3-(o-tolyl)-5-(o-trifluormethyl-phenyl)-1H-1,2,4-triazol,
1-Aethyl-3-(o-fluorphenyl)-5-(o-trifluormethyl-phenyl)-1H-1,2,4-triazol,
3-(o,o'-Dichlorphenyl)-1-methyl-5-(o-trifluormethyl-phenyl)-1H-1,2,4-triazol,
3-(o-Anisyl)-1-methyl-5-(o-trifluormethyl-phenyl)-1H-1,2,4-triazol,
3-(2,5-Dichlorphenyl)-1-methyl-5-(o-trifluormethyl-phenyl)-1H-1,2,4-triazol,
1-Methyl-3-(3-pyridyl)-5-(o-trifluormethyl-phenyl)-1H-1,2,4-triazol,
3-(o-Chlorphenyl)-1-(2,2,2-trifluoräthyl)-5-(o-trifluormethyl-phenyl)-1H-1,2,4-triazol,
1-Methyl-3-(2-pyridyl)-5-(o-trifluormethyl-phenyl)-1H-1,2,4-triazol,
1-Methyl-3-(4-pyridyl)-5-(o-trifluormethyl-phenyl)-1H-1,2,4-triazol,
3-(o-Jodphenyl)-1-methyl-5-(o-trifluormethyl-phenyl)-1H-1,2,4-triazol,
3-(o,p-Difluorphenyl)-1-methyl-5-(o-trifluormethyl-phenyl)-1H-1,2,4-triazol,
3-(4-Chlor-2-fluorphenyl)-1-methyl-5-(o-trifluormethyl-phenyl)-1H-1,2,4-triazol,
3-(3-Chlor-2-fluorphenyl)-1-methyl-5-(o-trifluormethyl-phenyl)-1H-1,2,4-triazol,
3-(2,3-Dichlorphenyl)-1-methyl-5-(o-trifluormethyl-phenyl)-1H-1,2,4-triazol,
3-(o-Cyanophenyl)-1-methyl-5-(o-trifluormethyl-phenyl)-1H-1,2,4-triazol und
1-(2-Chloräthyl)-3-(o-chlorphenyl)-5-(o-trifluormethyl-phenyl)-1H-1,2,4-triazol
ausgewählten Verbindung sowie Formulierungshilfsstoffe enthält.

9. Schädlingsbekämpfungsmittel nach Anspruch 1, dadurch gekennzeichnet, dass es eine wirksame Menge mindestens einer aus der Gruppe
1-Aethyl-3-(o,o'-difluorphenyl)-5-(o-trifluormethyl-phenyl)-1H-1,2,4-triazol und
3-(o-Chlorphenyl)-1-methyl-5-(4-trifluormethyl-3-pyridyl)-1H-1,2,4-triazol
ausgewählten Verbindung sowie Formulierungshilfsstoffe enthält.

10. Verfahren zur Herstellung von Verbindungen der allgemeinen Formel I gemäss Anspruch 1 und von ihren Säureadditionssalzen, dadurch gekennzeichnet, dass man

a) ein 1,2,4-Triazol der allgemeinen Formel

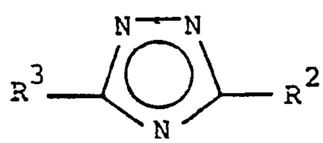

II

worin $R^2$ und $R^3$ die in Anspruch 1 angegebenen Bedeutungen besitzen, mit einer Verbindung der allgemeinen Formel

$$R^1 - X \qquad III$$

worin $R^1$ die in Anspruch 1 angegebene Bedeutung besitzt und X eine Abgangsgruppe bedeutet, umsetzt,

b) eine Verbindung der allgemeinen Formel

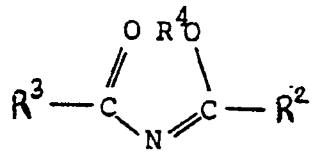

IV

worin $R^2$ und $R^3$ die in Anspruch 1 angegebenen Bedeutungen besitzen, und $R^4$ einen Niederalkylrest bedeutet,
oder ein Säureadditionssalz davon, mit einem Hydrazin der allgemeinen Formel

$$R^1-NH-NH_2 \qquad V$$

worin $R^1$ die in Anspruch 1 angegebene Bedeutung besitzt, umsetzt,
c) zwecks Herstellung derjenigen Verbindungen der Formel I, in denen $R^1$ $C_{1-4}$-Alkyl oder $C_{2-4}$-Alkenyl bedeutet, einen Imidsäureester der allgemeinen Formel

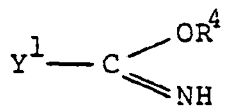

VI

worin $Y^1$ für $R^3$ oder für $R^2$ steht und $R^4$ und $R^2$ bzw. $R^3$ die in Anspruch 1 angegebenen Bedeutungen besitzen,
oder ein Säureadditionssalz davon, mit einem Säurehydrazid der allgemeinen Formel

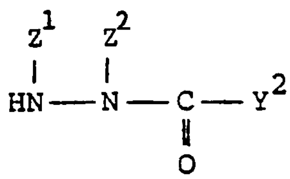

VII

worin $Y^2$ für $R^2$, $Z^1$ für $C_{1-4}$-Alkyl oder $C_{2-4}$-Alkenyl und $Z^2$ für Wasserstoff stehen (falls $Y^1$ der Formel VI $R^3$ bedeutet) oder $Y^2$ für $R^3$, $Z^1$ für Wasserstoff und $Z^2$ für $C_{1-4}$-Alkyl oder $C_{2-4}$-Alkenyl stehen (falls $Y^1$ der Formel VI $R^2$ bedeutet), wobei $R^2$ bzw. $R^3$ die in Anspruch 1 angegebene Bedeutung besitzt,
zu einem Acylamidrazon umsetzt und dieses durch Erhitzen cyclisiert,
d) zwecks Herstellung derjenigen Verbindungen der Formel I, in denen $R^1$ $C_{1-4}$-Alkyl oder $C_{2-4}$-Alkenyl bedeutet, ein Diacyl-, Monothiodiacyl- bzw. Dithiodiacylamin der allgemeinen Formel

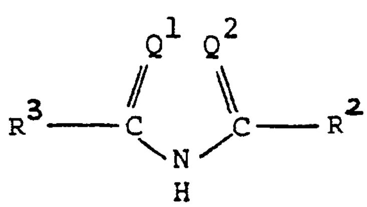

VIII

worin $R^2$ und $R^3$ die in Anspruch 1 angegebenen Bedeutungen besitzen und $Q^1$ und $Q^2$ unabhängig voneinander Sauerstoff oder Schwefel bedeuten,
mit einem Hydrazin der allgemeinen Formel

$$R^{1'}\text{-NH-NH}_2 \qquad V'$$

worin $R^{1'}$ $C_{1-4}$-Alkyl oder $C_{2-4}$-Alkenyl bedeutet, umsetzt, oder
e) zwecks Herstellung derjenigen Verbindungen der Formel I, in denen $R^1$ $C_{1-4}$-Halogenalkyl bedeutet, ein 1-Hydroxyalkyl-1H-1,2,4-triazol der allgemeinen Formel

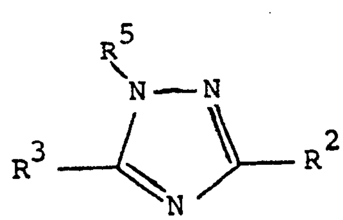

IX

worin $R^5$ $C_{1-4}$-Hydroxyalkyl bedeutet, und $R^2$ und $R^3$ die in Anspruch 1 angegebenen Bedeutungen besitzen,

mit einem Halogenierungsmittel behandelt, und erwünschtenfalls eine so erhaltene Verbindung der Formel I durch Umsetzung mit einer Säure in das entsprechende Säureadditionssalz überführt.

11. Verfahren nach Anspruch 10 zur Herstellung von denjenigen Verbindungen der Formel I, die in Anspruch 2 definiert sind.

12. Verfahren zur Herstellung eines Schädlingsbekämpfungsmittels, dadurch gekennzeichnet, dass man mindestens eine der in Anspruch 1 genannten Verbindungen mit Formulierungshilfsstoffen vermischt.

13. Verfahren zur Bekämpfung von Schädlingen, dadurch gekennzeichnet, dass man das zu schützende Gut oder die Schädlinge selbst mit einer wirksamen Menge mindestens einer in einem der Ansprüche 1, 6 und 9 genannten Verbindung bzw. eines Mittels gemäss einem dieser Ansprüche behandelt

14. Verfahren zur Bekämpfung von Schädlingen, dadurch gekennzeichnet, dass man das zu schützende Gut oder die Schädlinge selbst mit einer wirksamen Menge mindestens einer in einem der Ansprüche 2 bis 5, 7 und 8 genannten Verbindung bzw. eines Mittels gemäss eine dieser Ansprüche behandelt.

15. Verwendung einer in eine der Ansprüche 1, 6 und 9 genannten Verbindung bzw. eines Mittels gemäss einem dieser Ansprüche zur Bekämpfung von Schädlingen.

16. Verwendung einer in einem der Ansprüche 2 bis 5, 7 und 8 genannten Verbindung bzw. eines Mittels gemäss einem dieser Ansprüche zur Bekämpfung von Schädlingen.

## Claims

### Claims for the Contracting States : BE, CH, DE, FR, GB, IT, LI, NL, SE

1. Compounds of the general formula

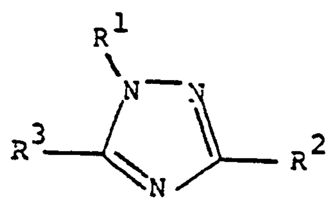

I

wherein

$R^1$ signifies $C_{1-4}$-alkyl, $C_{1-4}$-haloalkyl or $C_{2-4}$-alkenyl,

$R^2$ signifies phenyl which is substituted with 1 to 3 chlorine, bromine and/or iodine atoms, 1 to 5 fluorine atoms, 1 or 2 $C_{1-4}$-alkyl groups, 1 or 2 halomethyl groups, 1 or 2 $C_{1-2}$-alkoxy groups, 1 or 2 $C_{1-2}$-haloalkoxy groups, a methylthio group, a cyano group and/or a nitro group or 2-, 3- or 4-pyridyl which is optionally substituted with 1 to 3 chlorine atoms and/or a methyl group and

$R^3$ signifies o-trifluoromethyl-phenyl or 4-trifluoromethyl-3-pyridyl, and acid addition salts of the compounds of formula I.

2. Compounds according to claim 1, wherein $R^2$ signifies phenyl which is substituted with 1 to 3 fluorine, chlorine, bromine and/or iodine atoms, 1 or 2 $C_{1-4}$-alkyl groups, 1 or 2 trifluoromethyl groups, 1 or 2 $C_{1-2}$-alkoxy groups, a methylthio group, a cyano group and/or a nitro group or 2-, 3- or 4-pyridyl which is optionally substituted with 1 to 3 chlorine atoms and/or a methyl group and $R^3$ signifies o-trifluoromethyl-phenyl.

3. Compounds according to claim 1 or 2, wherein $R^1$ signifies $C_{1-4}$-alkyl.

4. Compounds according to any one of claims 1 to 3, wherein $R^2$ signifies substituted phenyl which has at least one substituent in an ortho-position.

5. Compounds according to claim 4, wherein $R^2$ signifies o-halophenyl or o,o'-dihalophenyl.

6. Compounds according to claim 4, wherein $R^2$ signifies o,p-dihalophenyl,

7. A compound according to claim 2, selected from :

3-(o-Chlorophenyl)-1-methyl-5-(o-trifluoromethyl-phenyl)-1H-1,2,4-triazole,
3-(o,o'-difluorophenyl)-1-methyl-5-(o-trifluoroethyl-phenyl)-1H-1,2,4-triazole,
3-(o-bromophenyl)-1-methyl-5-(o-trifluoromethyl-phenyl)-1H-1,2,4-triazole,
3-(2-chloro-6-fluorophenyl)-1-methyl-5-(o-trifluoromethyl-phenyl)-1H-1,2,4-triazole,
3-(o,p-dichlorophenyl)-1-methyl-5-(o-trifluoromethyl-phenyl)-1H-1,2,4-triazole and
3-(2-chloro-4-fluorophenyl)-1-methyl-5-(o-trifluoro-methyl-phenyl)-1H-1,2,4-triazole.

8. A compound according to claim 2, selected from :
3-(o-Fluorophenyl)-1-methyl-5-(o-trifluoromethyl-phenyl)-1H-1,2,4-triazole,
1-ethyl-3-(o-chlorophenyl)-5-(o-trifluoromethyl-phenyl)-1H-1,2,4-triazole,
1-allyl-3-(o-chlorophenyl)-5-(o-trifluoromethyl-phenyl)-1H-1,2,4-triazole,
3-(o-chlorophenyl)-1-(1-propenyl)-5-(o-trifluoromethyl-phenyl)-1H-1,2,4-triazole,
3-(o-chlorophenyl)-1-(n-propyl)-5-(o-trifluoromethyl-phenyl)-1H-1,2,4-triazole,
3-(o-chlorophenyl)-1-isopropyl-5-(o-trifluoromethyl-phenyl)-1H-1,2,4-triazole,
1-methyl-3,5-di(o-trifluoromethyl-phenyl)-1H-1,2,4-triazole,
1-methyl-3-(o-tolyl)-5-(o-trifluoromethyl-phenyl)-1H-1,2,4-triazole,
1-ethyl-3-(o-fluorophenyl)-5-(o-trifluoromethyl-phenyl)-1H-1,2,4-triazole,
3-(o,o'-dichlorophenyl)-1-methyl-5-(o-trifluoromethyl-phenyl)-1H-1,2,4-triazole,
3-(o-anisyl)-1-methyl-5-(o-trifluoromethyl-phenyl)-1H-1,2,4-triazole,
3-(2,5-dichlorophenyl)-1-methyl-5-(o-trifluoromethyl-phenyl)-1H-1,2,4-triazole,
1-methyl-3-(3-pyridyl)-5-(o-trifluoromethyl-phenyl)-1H-1,2,4-triazole,
3-(o-chlorophenyl)-1-(2,2,2-trifluoroethyl)-5-(o-trifluoromethyl-phenyl)-1H-1,2,4-triazole,
1-methyl-3-(2-pyridyl)-5-(o-trifluoromethyl-phenyl)-1H-1,2,4-triazole,
1-methyl-3-(4-pyridyl)-5-(o-trifluoromethyl-phenyl)-1H-1,2,4-triazole,
3-(o-iodophenyl)-1-methyl-5-(o-trifluoromethyl-phenyl)-1H-1,2,4-triazole,
3-(o,p-difluorophenyl)-1-methyl-5-(o-trifluoromethyl-phenyl)-1H-1,2,4-triazole,
3-(4-chloro-2-fluorophenyl)-1-methyl-5-(o-trifluoromethyl-phenyl)-1H-1,2,4-triazole,
3-(3-chloro-2-fluorophenyl)-1-methyl-5-(o-trifluoromethyl-phenyl)-1H-1,2,4-triazole,
3-(2,3-dichlorophenyl)-1-methyl-5-(o-trifluoromethyl-phenyl)-1H-1,2,4-triazole,
3-(o-cyanophenyl)-1-methyl-5-(o-trifluoromethyl-phenyl)-1H-1,2,4-triazole and
1-(2-chloroethyl)-3-(o-chlorophenyl)-5-(o-trifluoromethyl-phenyl)-1H-1,2,4-triazole.

9. A compound according to claim, selected from :
1-Ethyl-3-(o,o'-difluorophenyl)-5-(o-trifluoromethyl-phenyl)-1H-1,2,4-triazole and
3-(o-chlorophenyl)-1-methyl-5-(4-trifluoromethyl-3-pyridyl)-1H-1,2,4-triazole.

10. Compounds according to claim 1 as active ingredients of pest control compositions.

11. A pest control composition, characterized in that it contains an effective amount of at least one compound of general formula I in accordance with claim 1 or of an acid addition salt thereof as well as formulation adjuvants.

12. A pest control composition according to claim 11, characterized in that it contains an effective amount of at least one compound in accordance with claim 2 as well as formulation adjuvants.

13. A pest control composition according to claim 11, characterized in that it contains an effective amount of at least one compound in accordance with claim 7 as well as formulation adjuvants.

14. A process for the manufacture of compounds of general formula I in accordance with claim 1 and of their acid addition salts, characterized by

a) reacting a 1,2,4-triazole of the general formula

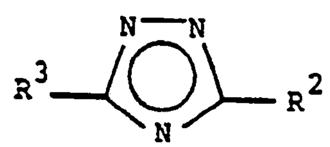

II

wherein $R^2$ and $R^3$ have the significances given in claim 1, with a compound of the general formula

$R^1$-X    III

wherein $R^1$ has the significance given in claim 1 and X signifies a leaving group,

b) reacting a compound of the general formula

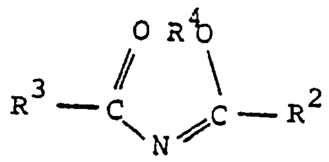

IV

wherein $R^2$ and $R^3$ have the significances given in claim 1 and $R^4$ signifies a lower alkyl residue, or an acid addition salt thereof with a hydrazine of the general formula

$$R^1\text{-NH-NH}_2 \qquad \text{V}$$

wherein $R^1$ has the significance given in claim 1,
c) for the manufacture of those compounds of formula I in which $R^1$ signifies $C_{1-4}$-alkyl or $C_{2-4}$-alkenyl, reacting an imidic ester of the general formula

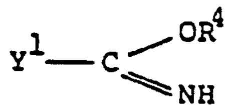

VI

wherein $Y^1$ stands for $R^3$ or for $R^2$ and $R^4$ and $R^2$ or $R^3$ have the significances given in claim 1, or an acid addition salt thereof with an acid hydrazide of the general formula

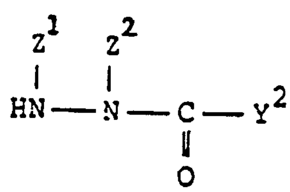

VII

wherein $Y^2$ stands for $R^2$, $Z^1$ stands for $C_{1-4}$- alkyl or $C_{2-4}$-alkenyl and $Z^2$ stands for hydrogen (when $Y^1$ in formula VI signifies $R^3$) or $Y^2$ stands for $R^3$, $Z^1$ stands for hydrogen and $Z^2$ stands for $C_{1-4}$-alkyl or $C_{2-4}$-alkenyl (when $Y^1$ in formula VI signifies $R^2$), whereby $R^2$ or $R^3$ has the significance given in claim 1, to an acylamidrazone and cyclizing this by heating,
d) for the manufacture of those compounds of formula I in which $R^1$ signifies $C_{1-4}$-alkyl or $C_{2-4}$-alkenyl, reacting a diacyl-, monothiodiacyl- or dithiodiacylamine of the general formula

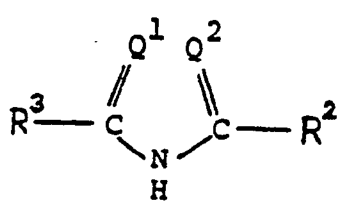

VIII

wherein $R^2$ and $R^3$ have the significances given in claim 1 and $Q^1$ and $Q^2$ each individually signify oxygen or sulphur, with a hydrazine of the general formula

$$R^{1\prime}\text{-NH-NH}_2 \qquad \text{V}'$$

wherein $R^{1'}$ signifies $C_{1-4}$-alkyl or $C_{2-4}$-alkenyl, or
e) for the manufacture of those compounds of formula I in which $R^1$ signifies $C_{1-4}$-haloalkyl, treating a 1-hydroxyalkyl-1H-1,2,4-triazole of the general formula

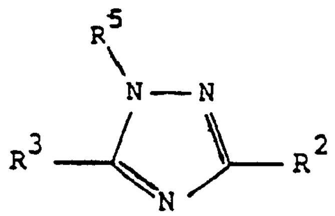

IX

wherein $R^5$ signifies $C_{1-4}$-hydroxyalkyl and $R^2$ and $R^3$ have the significances given in claim 1, with a halogenating agent, and, if desired, converting a thus-obtained compound of formula I by reaction with an acid into the corresponding acid addition salt.

15. A process according to claim 14 for the manufacture of compounds in accordance with claim 2.

16. A method for the control of pests, characterized by treating the locus to be protected or the pests themselves with an effective amount of a compound in accordance with any one of claims 1, 6 and 9 or of a composition in accordance with claim 11.

17. A method for the control of pests, characterized by treating the locus to be protected or the pests themselves with an effective amount of a compound in accordance with any one of claims 2 to 5, 7 and 8 or of a composition in accordance with claim 12 or 13.

18. The use of a compound in accordance with any one of claims 1, 6 and 9 or of a composition in accordance with claim for the control of pests.

19. The use of a compound in accordance with any one of claims 2 to 5, 7 and 8 or of a composition in accordance with claim 12 or 13 for the control of pests.

**Claim for the Contracting State : AT**

1. A pest control composition, characterized in that it contains an effective amount of at least one compound of the general formula

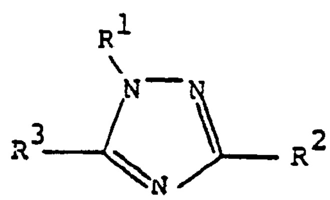

I

wherein
$R^1$ signifies $C_{1-4}$-alkyl, $C_{1-4}$-haloalkyl or $C_{2-4}$-alkenyl,
$R^2$ signifies phenyl which is substituted with 1 to 3 chlorine, bromine and/or iodine atoms, 1 to 5 fluorine atoms, 1 or 2 $C_{1-4}$-alkyl groups, 1 or 2 halomethyl groups, 1 or 2 $C_{1-2}$-alkoxy groups, 1 or 2 $C_{1-2}$-haloalkoxy groups, a methylthio group, a cyano group and/or a nitro group or 2-, 3- or 4-pyridyl which is optionally substituted with 1 to 3 chlorine atoms and/or a methyl group
and $R^3$ signifies o-trifluoromethyl-phenyl or 4-trifluoromethyl-3-pyridyl,
or of an acid addition salt thereof as well as formulation adjuvants.

2. A pest control composition according to claim 1, wherein $R^2$ signifies phenyl which is substituted with 1 to 3 fluorine, chlorine, bromine and/or iodine atoms, 1 or 2 $C_{1-4}$-alkyl groups, 1 or 2 trifluoromethyl groups, 1 or 2 $C_{1-2}$-alkoxy groups, a methylthio group, a cyano group and/or a nitro group or 2-, 3- or 4-pyridyl which is optionally substituted with 1 to 3 chlorine atoms and/or a methyl group and $R^3$ signifies o-trifluoromethyl-phenyl.

3. A pest control composition according to claim 1 or 2, wherein $R^1$ signifies $C_{1-4}$-alkyl.

4. A pest control composition according to any one of claims 1 to 3, wherein $R^2$ signifies substituted phenyl which has at least one substituent in an ortho-position.

5. A pest control composition according to claim 4, wherein $R^2$ signifies o-halophenyl or o,o'-dihalophenyl.

6. A pest control composition according to claim 4, wherein $R^2$ signifies o,p-dihalophenyl.

7. A pest control composition according to claim 2, characterized in that it contains an effective amount of at least one compound selected from the group
3-(o-chlorophenyl)-1-methyl-5-(o-trifluoromethyl-phenyl)-1H-1,2,4-triazole,
3-(o,o'-difluorophenyl)-1-methyl-5-(o-trifluoromethyl-phenyl)-1H-1,2,4-triazole,
3-(o-bromophenyl)-1-methyl-5-(o-trifluoromethyl-phenyl)-1H-1,2,4-triazole,
3-(2-chloro-6-fluorophenyl)-1-methyl-5-(o-trifluoromethyl-phenyl)-1H-1,2,4-triazole,
3-(o,p-dichlorophenyl)-1-methyl-5-(o-trifluoromethyl-phenyl)-1H-1,2,4-triazole and
3-(2-chloro-4-fluorophenyl)-1-methyl-5-(o-trifluoromethyl-phenyl)-1H-1,2,4-triazole
as well as formulation adjuvants.

8. A pest control composition according to claim 2, characterized in that it contains an effective amount of at least one compound selected from the group
3-(o-fluorophenyl)-1-methyl-5-(o-trifluoromethyl-phenyl)-1H-1,2,4-triazole,
1-ethyl-3-(o-chlorophenyl)-5-(o-trifluoromethyl-phenyl)-1H-1,2,4-triazole,
1-allyl-3-(o-chlorophenyl)-5-(o-trifluoromethyl-phenyl)-1H-1,2,4-triazole,
3-(o-chlorophenyl)-1-(-propenyl)-5-(o-trifluoromethyl-phenyl)-1H-1,2,4-triazole,
3-(o-chlorophenyl)-1-(n-propyl)-5-(o-trifluoromethyl-phenyl)-1H-1,2,4-triazole,
3-(o-chlorophenyl)-1-isopropyl-5-(o-trifluoromethyl-phenyl)-1H-1,2,4-triazole,
1-methyl-3,5-di(o-trifluoromethyl-phenyl)-1H-1,2,4-triazole,
1-methyl-3-(o-tolyl)-5-(o-trifluoromethyl-phenyl)-1H-1,2,4-triazole,
1-ethyl-3-(o-fluorophenyl)-5-(o-trifluoromethyl-phenyl)-1H-1,2,4-triazole,
3-(o,o'-dichlorophenyl)-1-methyl-5-(o-trifluoromethyl-phenyl)-1H-1,2,4-triazole,
3-(o-anisyl)-1-methyl-5-(o-trifluoromethyl-phenyl)-1H-1,2,4-triazole,
3-(2,5-dichlorophenyl)-1-methyl-5-(o-trifluoromethyl-phenyl)-1H-1,2,4-triazole,
1-methyl-3-(3-pyridyl)-5-(o-trifluoromethyl-phenyl)-1H-1,2,4-triazole,
3-(o-chlorophenyl)-1-(2,2,2-trifluoroethyl)-5-(o-trifluoromethyl-phenyl)-1H-1,2,4-triazole,
1-methyl-3-(2-pyridyl)-5-(o-trifluoromethyl-phenyl)-1H-1,2,4-triazole,
1-methyl-3-(4-pyridyl)-5-(o-trifluoromethyl-phenyl)-1H-1,2,4-triazole,
3-(o-iodophenyl)-1-methyl-5-(o-trifluoromethyl-phenyl)-1H-1,2,4-triazole,
3-(o,p-difluorophenyl)-1-methyl-5-(o-trifluoromethyl-phenyl)-1H-1,2,4-triazole,
3-(4-chloro-2-fluorophenyl)-1-methyl-5-(o-trifluoromethyl-phenyl)-1H-1,2,4-triazole,
3-(3-chloro-2-fluorophenyl)-1-methyl-5-(o-trifluoromethyl-phenyl)-1H-1,2,4-triazole,
3-(2,3-dichlorophenyl)-1-methyl-5-(o-trifluoromethyl-phenyl)-1H-1,2,4-triazole,
3-(o-cyanophenyl)-1-methyl-5-(o-trifluoromethyl-phenyl)-1H-1,2,4-triazole and
1-(2-chloroethyl)-3-(o-chlorophenyl)-5-(o-trifluoromethyl-phenyl)-1H-1,2,4-triazole
as well as formulation adjuvants.

9. A pest control composition according to claim 1, characterized in that it contains an effective amount of at least one compound selected from the group
1-ethyl-3-(o,o'-difluorophenyl)-5-(o-trifluoromethyl-phenyl)-1H-1,2,4-triazole and
3-(o-chlorophenyl)-1-methyl-5-(4-trifluoromethyl-3-pyridyl)-1H-1,2,4-triazole
as well as formulation adjuvants.

10. A process for the manufacture of compounds of general formula I in accordance with claim 1 and of their acid addition salts, characterized by

a) reacting a 1,2,4-triazole of the general formula

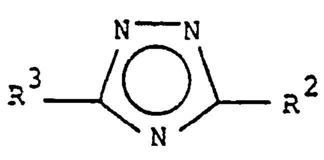

II

wherein $R^2$ and $R^3$ have the significances given in claim 1, with a compound of the general formula

$$R^1-X \qquad III$$

wherein $R^1$ has the significance given in claim 1 and X signifies a leaving group,

b) reacting a compound of the general formula

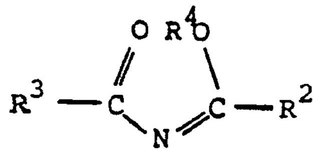

IV

wherein $R^2$ and $R^3$ have the significances given in claim 1 and $R^4$ signifies a lower alkyl residue, or an acid addition salt thereof with a hydrazine of the general formula

$$R^1\text{-NH-NH}_2 \qquad \text{V}$$

wherein $R^1$ has the significance given in claim 1,

c) for the manufacture of those compounds of formula I in which $R^1$ signifies $C_{1-4}$-alkyl or $C_{2-4}$-alkenyl, reacting an imidic ester of the general formula

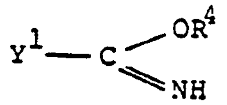

VI

wherein $Y^1$ stands for $R^3$ or for $R^2$ and $R^4$ and $R^2$ or $R^3$ have the significances given in claim 1, or an acid addition salt thereof with an acid hydrazide of the general formula

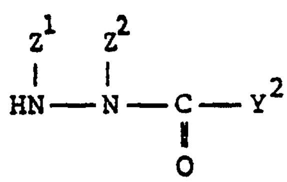

VII

wherein $Y^2$ stands for $R^2$, $Z^1$ stands for $C_{1-4}$-alkyl or $C_{2-4}$-alkenyl and $Z^2$ stands for hydrogen (when $Y^1$ in formula VI signifies $R^3$) or $Y^2$ stands for $R^3$, $Z^1$ stands for hydrogen and $Z^2$ stands for $C_{1-4}$-alkyl or $C_{2-4}$-alkenyl (when $Y^1$ in formula VI signifies $R^2$), whereby $R^2$ or $R^3$ has the significance given in claim 1, to an acylamidrazone and cyclizing this by heating,

d) for the manufacture of those compounds of formula I in which $R^1$ signifies $C_{1-4}$-alkyl or $C_{2-4}$-alkenyl, reacting a diacyl-, monothiodiacyl- or dithiodiacylamine of the general formula

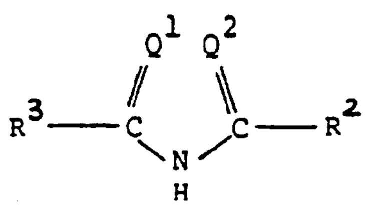

VIII

wherein $R^2$ and $R^3$ have the significances given in claim 1 and $Q^1$ and $Q^2$ each individually signify oxygen or sulphur, with a hydrazine of the general formula

$R^{1'}-NH-NH_2$ V'

wherein $R^{1'}$ signifies $C_{1-4}$-alkyl or $C_{2-4}$-alkenyl, or

e) for the manufacture of those compounds of formula I in which $R^1$ signifies $C_{1-4}$-haloalkyl, treating a 1-hydroxyalkyl-1H-1,2,4-triazole of the general formula

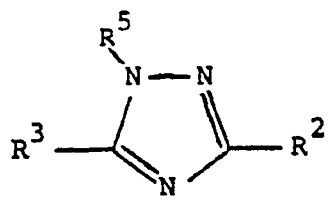

IX

wherein $R^5$ signifies $C_{1-4}$-hydroxyalkyl and $R^2$ and $R^3$ have the significances given in claim 1, with a halogenating agent, and, if desired, converting a thus-obtained compound of formula I by reaction with an acid into the corresponding acid addition salt.

11. A process according to claim 10 for the manufacture of those compounds of formula I which are defined in claim 2.

12. A process for the manufacture of a pest control composition, characterized by mixing at least one of the compounds set forth in claim 1 with formulation adjuvants.

13. A method for the control of pests, characterized by treating the locus to be protected or the pests themselves with an effective amount of at least one compound set forth in any one of claims 1, 6 and 9 or of a composition in accordance with any one of these claims.

14. A method for the control of pests, characterized by treating the locus to be protected or the pests themselves with an effective amount of at least one of the compounds set forth in claims 2 to 5, 7 and 8 or of a composition in accordance with any one of these claims.

15. The use of a compound set forth in any one of claims 1, 6 and 9 or of a composition in accordance with any one of these claims for the control of pests.

16. The use of a compound set forth in any one of claims 2 to 5, 7 and 8 or of a composition in accordance with any one of these claims for the control of pests.

## Revendications

### Revendications pour les Etats contractants : BE, CH, DE, FR, GB, IT, LI, NL, SE

1. Composés de formule générale

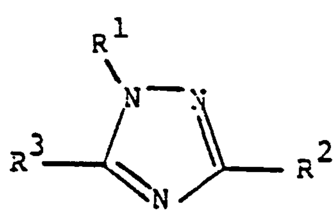

I

dans laquelle

$R^1$ représente un groupe alkyle en C 1-C 4, halogénoalkyle en C 1-C 4 ou alcényle en C 2-C 4,

$R^2$ représente un groupe phényle substitué par un à trois atomes de chlore, de brome et/ou d'iode, 1 à 5 atomes de fluor, 1 ou 2 groupes alkyle en C 1-C 4, un ou deux groupes halogénométhyle, un ou deux groupes alcoxy en C 1-C 2, un ou deux groupes halogénoalcoxy en C 1-C 2, un groupe méthylthio, un groupe cyano et/ou un groupe nitro, ou un groupe 2-, 3- ou 4-pyridyle éventuellement substitué par 1 à 3 atomes de chlore et/ou un groupe méthyle, et

$R^3$ représente un groupe o-trifluorométhyl-phényle ou 4-trifluorométhyl-3-pyridyle, ainsi que les sels des composés de formule I formés par addition avec des acides.

2. Composés selon la revendication 1, dans lesquels $R^2$ représente un groupe phényle substitué par 1 à 3 atomes de fluor, de chlore, de brome et/ou d'iode, un ou deux groupes alkyle en C 1-C 4, un ou deux groupes trifluorométhyle, un ou deux groupes alcoxy en C 1-C 2, un groupe méthylthio, un groupe cyano et/ou un groupe nitro, ou un groupe 2-, 3- ou 4-pyridyle éventuellement substitué par 1 à 3 atomes de chlore et/ou un groupe méthyle, et $R^3$ représente un groupe o-trifluorométhyl-phényle.

3. Composés selon la revendication 1 ou 2, dans lesquels $R^1$ représente un groupe alkyle en C 1-C 4.

4. Composés selon l'une des revendications 1 à 3, dans lesquels $R^2$ représente un groupe phényle substitué, portant au moins un substituant dans une position ortho.

5. Composés selon la revendication 4, dans lesquels $R^2$ représente un groupe o-halogénophényle ou o,o'-dihalogénophényle.

6. Composés selon la revendication 4, dans lesquels $R^2$ représente un groupe o,p-dihalogénophényle.

7. Un composé selon la revendication 2, choisi parmi les suivants :
3-(o-chlorophényl)-1-méthyl-5-(o-trifluorométhyl-phényl)-1H-1,2,4-triazole,
3-(o,o'-difluorophényl)-1-méthyl-5-(o-trifluorométhyl-phényl)-1H-1,2,4-triazole,
3-(o-bromophényl)-1-méthyl-5-(o-trifluorométhyl-phényl)-1H-1,2,4-triazole,
3-(2-chloro-6-fluorophényl)-1-méthyl-5-(o-trifluorométhyl-phényl)-1H-1,2,4-triazole,
3-(o,p-dichlorophényl)-1-méthyl-5-(o-trifluorométhyl-phényl)-1H-1,2,4-triazole, et
3-(2-chloro-4-fluorophényl)-1-méthyl-5-(o-trifluorométhyl-phényl)-1H-1,2,4-triazole.

8. Un composé selon la revendication 2, choisi parmi les suivants :
3-(o-fluorophényl)-1-méthyl-5-(o-trifluorométhyl-phényl)-1H-1,2,4-triazole,
1-éthyl-3-(o-chlorophényl)-5-(o-trifluorométhyl-phényl)-1H-1,2,4-triazole,
1-allyl-3-(o-chlorophényl)-5-(o-trifluorométhyl-phényl)-1H-1,2,4-triazole,
3-(o-chlorophényl)-1-(1-propényl)-5-(o-trifluorométhyl-phényl)-1H-1,2,4-triazole,
3-(o-chlorophényl)-1-(n-propyl)-5-(o-trifluorométhyl-phényl)-1H-1,2,4-triazole,
3-(o-chlorophényl)-1-isopropyl)-5-(o-trifluorométhyl-phényl)-1H-1, 2,4-triazole,
1-méthyl-3,5-di-(o-trifluorométhyl-phényl)-1H-1,2,4-triazole,
1-méthyl-3-(o-tolyl)-5-(o-trifluorométhyl-phényl)-1H-1,2,4-triazole,
1-éthyl-3-(o-fluorophényl)-5-(o-trifluorométhyl-phényl)-1H-1,2,4-triazole,
3-(o,o'-dichlorophényl)-1-méthyl-5-(o-trifluorométhyl-phényl)-1H-1,2,4-triazole,
3-(o-anisyl)-1-méthyl-5-(o-trifluorométhyl-phényl)-1H-1,2,4-triazole,
3-(2,5-dichlorophényl)-1-méthyl-5-(o-trifluorométhyl-phényl)-1H-1,2,4-triazole,
1-méthyl-3-(3-pyridyl)-5-(o-trifluorométhyl-phényl)-1H-1,2,4-triazole,
3-(o-chlorophényl)-1-(2,2,2-trifluoréthyl)-5-(o-trifluorométhyl-phényl)-1H-1,2,4-triazole,
1-méthyl-3-(2-pyridyl)-5-(o-trifluorométhyl-phényl)-1H-1,2,4-triazole,
1-méthyl-3-(4-pyridyl)-5-(o-trifluorométhyl-phényl)-1H-1,2,4-triazole,
3-(o-iodophényl)-1-méthyl-5-(o-trifluorométhyl-phényl)-1H-1,2,4-triazole,
3-(o,p-difluorophényl)-1-méthyl-5-(o-trifluorométhyl-phényl)-1H-1,2,4-triazole,
3-(4-chloro-2-fluorophényl)-1-méthyl-5-(o-trifluorométhyl-phényl)-1H-1,2,4-triazole,
3-(3-chloro-2-fluorophényl)-1-méthyl-5-(o-trifluorométhyl-phényl)-1H-1,2,4-triazole,
3-(2,3-dichlorophényl)-1-méthyl-5-(o-trifluorométhyl-phényl)-1H-1,2,4-triazole,
3-(o-cyanophényl)-1-méthyl-5-(o-trifluorométhyl-phényl)-1H-1,2,4-triazole, et
1-(2-chloréthyl)-3-(o-chlorophényl)-5-(o-trifluorométhyl-phényl)-1H-1,2,4-triazole.

9. Un composé selon la revendication 1, choisi parmi les suivants :
1-éthyl-3-(o,o'-difluorophényl)-5-(o-trifluorométhyl-phényl)-1H-1,2,4-triazole, et
3-(o-chlorophényl)-1-méthyl-5-(4-trifluorométhyl-3-pyridyl)-1H-1,2,4-triazole.

10. Composés selon la revendication 1, en tant que substances actives de produits pesticides.

11. Produit pesticide, caractérisé en ce qu'il contient une quantité efficace d'au moins un composé de formule générale I selon la revendication 1 ou d'un sel d'un tel composé formé par addition avec un acide, avec des produits auxiliaires de formulation.

12. Produit pesticide selon la revendication 11, caractérisé en ce qu'il contient une quantité efficace d'au moins un composé selon la revendication 2, avec des produits auxiliaires de formulation.

13. Produit pesticide selon la revendication 11, caractérisé en ce qu'il contient une quantité efficace d'au moins un composé selon la revendication 7.

14. Procédé de préparation des composés de formule générale I selon revendication 1, et de leurs sels formés par addition avec des acides, caractérisé en ce que :

a) on fait réagir un 1,2,4-triazole de formule générale

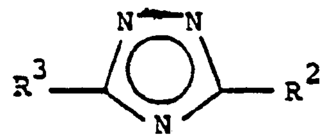

II

dans laquelle $R^2$ et $R^3$ ont les significations indiquées dans la revendication 1, avec un composé de formule générale

$$R^1 - X \qquad III$$

dans laquelle $R^1$ a les significations indiquées dans la revendication 1 et X représente un groupe éliminable,

b) on fait réagir un composé de formule générale

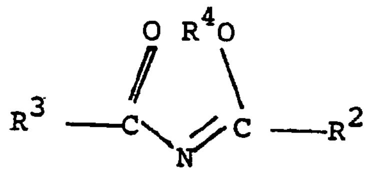

IV

dans laquelle $R^2$ et $R^3$ ont les significations indiquées dans la revendication 1, et $R^4$ représente un groupe alkyle inférieur, ou un sel d'un tel composé formé par addition avec un acide, avec une hydrazine de formule générale

$$R^1 - NH\text{-}NH_2 \qquad V$$

dans laquelle $R^1$ a les significations indiquées dans la revendication 1,

c) pour préparer les composés de formule I dans laquelle $R^1$ représente un groupe alkyle en C 1-C 4 ou alcényle en C 2-C 4, on fait réagir un ester d'imidoacide de formule générale

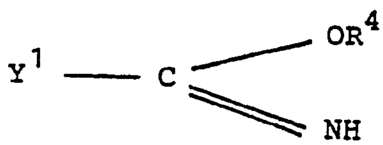

VI

dans laquelle $Y^1$ représente $R^3$ ou $R^2$ et $R^4$, $R^2$ et $R^3$ ont les significations indiquées dans la revendication 1, ou un sel d'un tel composé formé par addition avec un acide, avec un hydrazide de formule générale

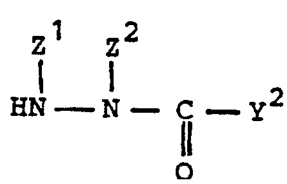

VII

dans laquelle $Y^2$ représente $R^2$, $Z^1$ représente un groupe alkyle en C 1-C 4 ou alcényle en C 2-C 4 et $Z^2$ représente l'hydrogène (lorsque le symbole $Y^1$ de la formule VI représente $R^3$) ou bien $Y^2$ représente $R^3$, $Z^1$ représente l'hydrogène et $Z^2$ représente un groupe alkyle en C 1-C 4 ou alcényle en C 2-C 4 (lorsque le symbole $Y^1$ de la formule VI représente $R^2$), $R^2$ et $R^3$ ayant les significations indiquées dans la revendication 1, ce qui donne une acylamidrazone qu'on cyclise par chauffage,

d) pour préparer les composés de formule I dans laquelle $R^1$ représente un groupe alkyle en C 1-C 4 ou alcényle en C 2-C 4, on fait réagir une diacyl-, une monothiodiacyl- ou respectivement une dithiodiacylamine de formule générale

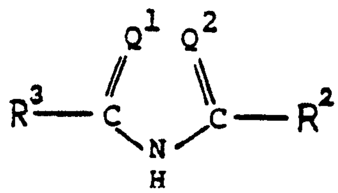

VIII

dans laquelle $R^2$ et $R^3$ ont les significations indiquées dans la revendication 1, et $Q^1$ et $Q^2$ représentent chacun, indépendamment l'un de l'autre, l'oxygène ou le soufre, avec une hydrazine de formule générale

$$R^{1'}\text{-NH-NH}_2 \qquad V'$$

dans laquelle $R^{1'}$ représente un groupe alkyle en C 1-C 4 ou alcényle en C 2-C 4, ou bien

e) pour préparer les composés de formule I dans laquelle $R^1$ représente un groupe halogénoalkyle en C 1-C 4, on traite un 1-hydroxyalkyl-1H-1,,4-triazole de formule générale

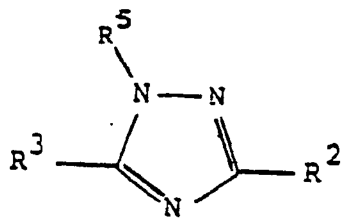

IX

dans laquelle $R^5$ représente un groupe hydroxyalkyle en C 1-C 4 et $R^2$ et $R^3$ ont les significations indiquées dans la revendication 1, par un agent halogénant, et si on le désire, on convertit un composé ainsi obtenu et répondant à la formule I, par réaction avec un acide, en le sel correspondant formé par addition.

15. Procédé selon la revendication 14, pour la préparation des composés selon la revendication 2.

16. Procédé pour combattre les parasites, caractérisé en ce que l'on traite la matière à protéger ou les parasites eux-mêmes par une quantité efficace d'un composé selon l'une des revendications 1, 6 et 9, ou d'un produit selon la revendication 11.

17. Procédé pour combattre les parasites, caractérisé en ce que l'on traite la matière à protéger ou les parasites eux-mêmes par une quantité efficace d'un composé selon l'une des revendications 2 à 5, 7 et 8, ou d'un produit selon la revendication 12 ou 13.

18. Utilisation d'un composé selon l'une des revendications 1, 6 et 9, ou d'un produit selon la revendication 11, pour la lutte contre les parasites.

19. Utilisation d'un composé selon l'une des revendications 2 à 5, 7 et 8, ou d'un produit selon la revendication 12 ou 13, pour combattre les parasites.

**Revendication pour l'Etat contractant : AT**

1. Produit pesticide, caractérisé en ce qu'il contient une quantité efficace d'au moins un composé de formule générale

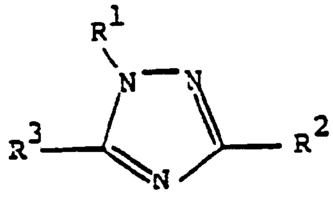

I

dans laquelle

$R^1$ représente un groupe alkyle en C 1-C 4, halogénoalkyle en C 1-C 4 ou alcényle en C 2-C 4,

$R^2$ représente un groupe phényle substitué par 1 à 3 atomes de chlore, de brome et/ou d'iode, 1 à 5 atomes de fluor, un ou deux groupes alkyle en C 1-C 4, un ou deux groupes halogénométhyle, un ou deux groupes alcoxy en C 1-C 2, un ou deux groupes halogénoalcoxy en C 1-C 2, un groupe méthylthio, un groupe cyano et/ou un groupe nitro, ou un groupe 2-, 3- ou 4-pyridyle éventuellement substitué par 1 à 3 atomes de chlore et/ou un groupe méthyle, et

$R^3$ représente un groupe o-trifluorométhyl-phényle ou 4-trifluorométhyl-3-pyridyle, ou d'un sel d'un tel composé formé par addition avec un acide, ainsi que des produits auxiliaires de formulation.

2. Produit pesticide selon la revendication 1, pour lequel $R^2$ représente un groupe phényle substitué par 1 à 3 atomes de fluor, de chlore, de brome et/ou d'iode, un ou deux groupes alkyle en C 1-C 4, un ou deux groupes trifluorométhyle, un ou deux groupes alcoxy en C 1-C 2, un groupe méthylthio, un groupe cyano et/ou un groupe nitro, ou un groupe 2-, 3- ou 4-pyridyle éventuellement substitué par 1 à 3 atomes de chlore et/ou un groupe méthyle, et $R^3$ représente un groupe o-trifluorométhyl-phényle.

3. Produit pesticide selon la revendication 1 ou 2, pour lequel $R^1$ représente un groupe alkyle en C 1-C 4.

4. Produit pesticide selon l'une des revendications 1 à 3, pour lequel $R^2$ représente un groupe phényle substitué, portant au moins un substituant dans une position ortho.

5. Produit pesticide selon la revendication 4, pour lequel $R^2$ représente un groupe o-halogénophényle ou o,o'-dihalogénophényle.

6. Produit pesticide selon la revendication 4, pour lequel $R^2$ représente un groupe o,p-dihalogénophényle.

7. Produit pesticide selon la revendication 2, caractérisé en ce qu'il contient une quantité efficace d'au moins un composé du groupe suivant :
3-(o-chlorophényl)-1-méthyl-5-(o-trifluorométhyl-phenyl)-1H-1,2,4-triazole,
3-(o,o'-difluorophényl)-1-méthyl-5-(o-trifluorométhyl-phényl)-1H-1,2,4-triazole,
3-(o-bromophényl)-1-méthyl-5-(o-trifluorométhyl-phényl)-1H-1,2,4-triazole,
3-(2-chloro-6-fluorophényl)-1-méthyl-5-(o-trifluorométhyl-phényl)-1H-1,2,4-triazole,
3-(o,p-dichlorophényl)-1-méthyl-5-(o-trifluorométhyl-phényl)-1H-1,2,4-triazole, et
3-(2-chloro-4-fluorophényl)-1-méthyl-5-(o-trifluorométhyl-phényl)-1H-1,2,4-triazole,
avec des produits auxiliaires de formulation.

8. Produit pesticide selon la revendication 2, caractérisé en ce qu'il contient une quantité efficace d'au moins un composé du groupe suivant :
3-(o-fluorophényl)-1-méthyl-5-(o-trifluorométhyl-phényl)-1H-1,2,4-triazole,
1-éthyl-3-(o-chlorophényl)-5-(o-trifluorométhyl-phényl)-1H-1,2,4-triazole,
1-allyl-3-(o-chlorophényl)-5-(o-trifluorométhyl-phényl)-1H-1,2,4-triazole,
3-(o-chlorophényl)-1-(1-propényl)-5-(o-trifluorométhyl-phényl)-1H-1,2,4-triazole,
3-(o-chlorophényl)-1-(n-propyl)-5-(o-trifluorométhyl-phényl)-1H-1,2,4-triazole,
3-(o-chlorophényl)-1-isopropyl-5-(o-trifluorométhyl-phényl)-1H-1,2,4-triazole,
1-méthyl-3,5-di-(o-trifluorométhyl-phényl)-1H-1,2,4-triazole,
1-méthyl-3-(o-tolyl)-5-(o-trifluorométhyl-phényl)-1H-1,2,4-triazole,
1-éthyl-3-(o-fluorophényl)-5-(o-trifluorométhyl-phényl)-1H-1,2,4-triazole,
3-(o,o'-dichlorophényl)-1-méthyl-5-(o-trifluorométhyl-phényl)-1H-1,2,4-triazole,
3-(o-anisyl)-1-méthyl-5-(o-trifluorométhyl-phényl)-1H-1,2,4-triazole,
3-(2,5-dichlorophényl)-1-methyl-5-(o-trifluorométhyl-phényl)-1H-1,2,4-triazole,
1-méthyl-3-(3-pyridyl)-5-(o-trifluorométhyl-phényl)-1H-1,2,4-triazole,
3-(o-chlorophényl)-1-(2,2,2-trifluoréthyl)-5-(o-trifluorométhyl-phényl)-1H-1,2,4-triazole,
1-méthyl-3-(2-pyridyl)-5-(o-trifluoromethyl-phényl)-1H-1,2,4-triazole,
1-méthyl-3-(4-pyridyl)-5-(o-trifluorométhyl-phényl)-1H-1,2,4-triazole,
3-(o-iodophényl)-1-méthyl-5-(o-trifluorométhyl-phényl)-1H-1,2,4-triazole,
3-(o,p-difluorophényl)-1-méthyl-5-(o-trifluorométhyl-phényl)-1H-1,2,4-triazole,
3-(4-chloro-2-fluorophényl)-1-méthyl-5-(o-trifluorométhyl-phényl)-1H-1,2,4-triazole,
3-(3-chloro-2-fluorophényl)-1-méthyl-5-(o-trifluorométhyl-phényl)-1H-1,2,4-triazole,
3-(2,3-dichlorophényl)-1-méthyl-5-(o-trifluorométhyl-phényl)-1H-1,2,4-triazole,
3-(o-cyanophényl)-1-méthyl-5-(o-trifluorométhyl-phényl)-1H-1,2,4-triazole, et
1-(2-chloréthyl)-3-(o-chlorophényl)-5-(o-trifluorométhyl-phényl)-1H-1,2,4-triazole,
avec des produits auxiliaires de formulation.

9. Produit pesticide selon la revendication 1, caractérisé en ce qu'il contient une quantité efficace d'au moins un composé du groupe suivant :
1-éthyl-3-(o,o'-difluorophényl)-5-(o-trifluorométhyl-phényl)-1H-1,2,4-triazole, et

3-(o-chlorophényl)-1-méthyl-5-(4-trifluorométhyl-3-pyridyl)-1H-1,2,4-triazole, avec des produits auxiliaires de formulation.

10. Procédé de préparation des composés de formule générale I de la revendication 1, et de leurs sels formés par addition avec des acides, caractérisé en ce que :

a) on fait réagir un 1,2,4-triazole de formule générale

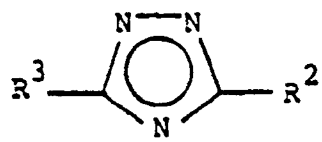

II

dans laquelle $R^2$ et $R^3$ ont les significations indiquées dans la revendication 1, avec un composé de formule générale

$$R^1 - X \qquad III$$

dans laquelle $R^1$ a les significations indiquées dans la revendication 1, et X représente un groupe éliminable,

b) on fait réagir un composé de formule générale

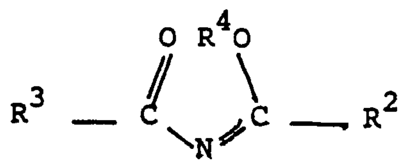

IV

dans laquelle $R^2$ et $R^3$ ont les significations indiquées dans la revendication 1, et $R^4$ représente un groupe alkyle inférieur, ou un sel d'un tel composé formé par addition avec un acide, avec une hydrazine de formule générale

$$R^1 - NH - NH_2 \qquad V$$

dans laquelle $R^1$ a les significations indiquées dans la revendication 1,

c) pour préparer les composés de formule I dans laquelle $R^1$ représente un groupe alkyle en C 1-C 4 ou alcényle en C 2-C 4, on fait réagir un ester d'imidoacide de formule générale

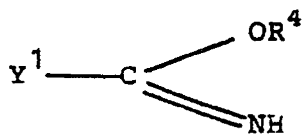

VI

dans laquelle $Y^1$ représente $R^3$ ou $R^2$, et $R^4$, $R^2$ et $R^3$ ont les significations indiquées dans la revendication 1, ou un sel d'un tel composé formé par addition avec un acide, avec un hydrazide de formule générale

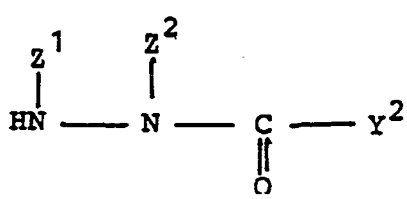

VII

dans laquelle $Y^2$ représente $R^2$, $Z^1$ représente un groupe alkyle en C 1-C 4 ou alcényle en C 2-C 4 et $Z^2$ représente l'hydrogène (lorsque le symbole $Y^1$ de la formule VI représente $R^3$) ou bien $Y^2$ représente

$R^3$, $Z^1$ représente l'hydrogène et $Z^2$ un groupe alkyle en C 1-C 4 ou alcényle en C 2-C 4 (lorsque le symbole $Y^1$ de la formule VI représente $R^2$), $R^2$ et $R^3$ ayant respectivement les significations indiquées dans la revendication 1, ce qui donne une acylamidrazone qu'on cyclise par chauffage,

d) pour préparer les composés de formule I dans laquelle $R^1$ représente un groupe alkyle en C 1-C 4 ou alcényle en C 2-C 4, on fait réagir une diacyl-, une monothiodiacyl- ou respectivement une dithiodiacylamine de formule générale

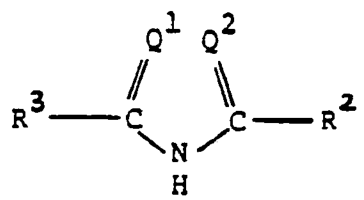

VIII

dans laquelle $R^2$ et $R^3$ ont les significations indiquées dans la revendication 1, et $Q^1$ et $Q^2$ représentent chacun, indépendamment l'un de l'autre, l'oxygène ou le soufre, avec une hydrazine de formule générale

$$R^{1'} - NH - NH_2 \qquad V'$$

dans laquelle $R^{1'}$ représente un groupe alkyle en C 1-C 4 ou alcényle en C 2-C 4, ou bien

e) pour préparer les composés de formule I dans laquelle $R^1$ représente un groupe halogénoalkyle en C 1-C 4, on traite un 1-hydroxyalkyl-1H-1,2,4-triazole de formule générale

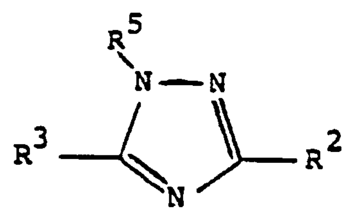

IX

dans laquelle $R^5$ représente un groupe hydroxyalkyle en C 1-C 4 et $R^2$ et $R^3$ ont les significations indiquées dans la revendication 1, par un agent halogénant, et si on le désire, on convertit un composé ainsi obtenu et répondant à la formule I en sel formé par addition par réaction avec l'acide correspondant.

11. Procédé selon la revendication 10, pour préparer les composés de formule I définis dans la revendication 2.

12. Procédé de préparation d'un produit pesticide, caractérisé en ce que l'on mélange au moins un des composés mentionnés dans la revendication 1 avec des produits auxiliaires de formulation.

13. Procédé pour combattre les parasites, caractérisé en ce que l'on traite la matière à protéger ou les parasites eux-mêmes par une quantité efficace d'au moins un composé mentionné dans l'une des revendications 1, 6 et 9, ou d'un produit selon l'une de ces revendications.

14. Procédé pour combattre les parasites, caractérisé en ce que l'on traite la matière à protéger ou les parasites eux-mêmes par une quantité efficace d'au moins un composé mentionné dans l'une des revendications 2 à 5, 7 et 8, ou d'un produit selon l'une de ces revendications.

15. Utilisation d'un composé mentionné dans l'une des revendications 1, 6 et 9, ou d'un produit selon l'une de ces revendications pour la lutte contre les parasites.

16. Utilisation d'un composé mentionné dans l'une des revendications 2 à 5, 7 et 8, ou d'un produit selon l'une de ces revendications dans la lutte contre les parasites.